# EUROPEAN PATENT SPECIFICATION

(11) **EP 3 730 476 B1**
(45) Date of publication and mention of the grant of the patent: **20.07.2022**
(21) Application number: 18890636.6
(22) Date of filing: 21.12.2018
(51) Int. Cl.: C07C 69/24, C07C 69/608, A61K 8/37, A61Q 1/00, C07C 69/96, C07C 69/74, A61Q 19/10, A61Q 19/00, A61Q 17/04, A61Q 17/02, A61Q 13/00, A61Q 5/02, A61Q 5/00, C11B 9/00, C11D 3/50

(54) **COMPOUND HAVING MUSK-LIKE SCENT AND PERFUME COMPOSITION CONTAINING SAME**
VERBINDUNG MIT MOSCHUSÄHNLICHEM GERUCH UND PARFÜMZUSAMMENSETZUNG DAMIT
COMPOSÉ AYANT UNE ODEUR DE TYPE MUSC ET COMPOSITION DE PARFUM LE CONTENANT

(30) Priority: 22.12.2017 JP 2017246076
(43) Date of publication of application: 28.10.2020
(73) Proprietor: Takasago International Corporation, Tokyo 144-8721 (JP)
(72) Inventor: SEKI Yuna, Hiratsuka-shi, Kanagawa 254-0073 (JP); YAMAMOTO Kenichi, Hiratsuka-shi, Kanagawa 254-0073 (JP); OTAKE Masaya, Hiratsuka-shi, Kanagawa 254-0073 (JP)
(74) Representative: Hoffmann Eitle
(86) International application number: PCT/JP2018/047171
(87) International publication number: WO 2019/124533

(56) References cited:
- WO-A2-03/082799
- JP-A- H0 672 952
- JP-A- 2011 037 761
- US-A1- 2013 261 036

## Description

### TECHNICAL FIELD

The present invention relates to a compound which can be preferred for use as a perfume material having a musk-like scent, and a perfume composition containing such a compound, as well as a product scented with such a perfume composition.

### BACKGROUND ART

The perfume industry constantly requires novel perfume materials which allow expansion and/or improvement of the range of aroma notes available to perfumers. Moreover, in recent years, with increase in the variety of products such as various perfumery and cosmetic products, sanitary materials and medicaments, there has been a greater demand than ever before for the development of perfume materials showing strong diffusibility, good biodegradability, high palatability, strong retention, good stability and high safety when used as perfumes for perfumery and cosmetic products and sanitary materials, further as perfumes for medicaments, etc.

In particular, as to perfume materials having a musk-like scent, nitromusk had been very often used until around 1990, but was restricted for use because nitromusk was pointed out to have the risk of causing bioaccumulation and acting on humans as a reproductive toxin or an endocrine disruptor, etc. After that, the need for its aroma quality per se has also risen, and it has been expected to develop a new perfume material.

As to perfume materials having a musk-like scent, for example, the compounds described in Patent Literatures 1 to 5 have been known.

### Citation List

### Patent Literature

Patent Literature 1: WO2005/108534
Patent Literature 2: WO2011/29895
Patent Literature 3: JP H06-72952 A
Patent Literature 4: WO2000/14051
Patent Literature 5: US Patent Application Publication No. 2004/53811

### SUMMARY OF INVENTION

### TECHNICAL PROBLEM

Under these circumstances, there has been a demand for the development of a novel perfume material. In particular, there has been a demand for the provision of a novel perfume material having a musk-like scent which satisfies the above requirements.

### SOLUTION TO PROBLEM

As a result of extensive and intensive efforts, the inventors of the present invention have succeeded in developing a novel perfume material which may have a musk-like scent. As far as we know, the compound of the present invention is a novel compound which has never been known before.

Namely, the present invention provides the compound shown below and a perfume composition containing the same, a product scented with the perfume composition, as well as a method for enhancing or modulating the scent of a perfume composition, which comprises adding the compound.
[1] A compound represented by the following general formula: 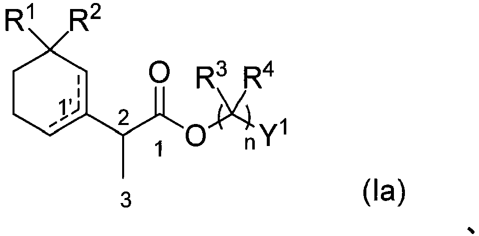 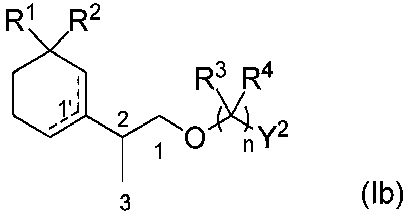 or 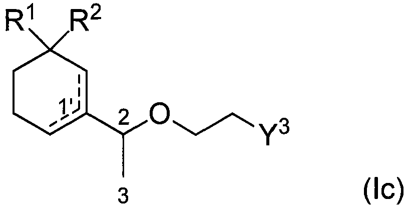 wherein
   R¹ and R² are each independently a hydrogen atom or a methyl group, n is 2 or 3,
   two or more R³ and R⁴ are each independently a hydrogen atom or a methyl group,
   Y¹ is -O-CO-Z, -O-CO-O-Z, -CO-Z, -CO-O-Z or -CH(Z¹)-CO-Z² , Y² is -O-CO-Z or -O-CO-O-Z, and Y³ is -O-CO-Z³, where Z is a linear or branched alkyl group containing 1 to 5 carbon atoms or a cycloalkyl group containing 3 to 5 carbon atoms, Z¹ is a hydrogen atom, a methyl group or an ethyl group, Z² is a linear or branched alkyl group containing 1 to 3 carbon atoms, and Z³ is a linear or branched alkyl group containing 1 to 5 carbon atoms or a cycloalkyl group containing 3 to 5 carbon atoms, and
   the adjacent two ring-constituting double lines, each consisting of a solid line and a dotted line, are each independently a double bond or a single bond, except for the case where both are double bonds.
[2] The compound according to [1] above, which is represented by the following general formula: 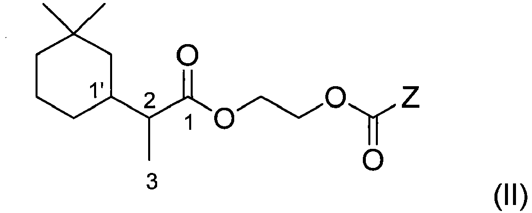 wherein Z is a linear or branched alkyl group containing 1 to 5 carbon atoms or a cycloalkyl group containing 3 to 5 carbon atoms.
[3] The compound according to [1] or [2] above, which is represented by the following structural formula. 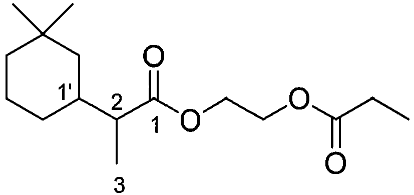
[4] The compound according to [3] above, wherein the relative configuration at the 1'- and 2-positions is (R^{∗},S^{∗}) or (R^{∗},R^{∗}) or a mixture of (R^{∗},S^{∗}) and (R^{∗},R^{∗}).
[5] The compound according to [3] above, wherein the relative configuration at the 1'- and 2-positions is (R^{∗},S^{∗}).
[6] A perfume composition containing at least one compound according to any one of [1] to [5] above.
[7] A musk perfume composition containing at least one compound according to any one of [1] to [5] above.
[8] A product scented with the perfume composition according to [6] or [7] above.
[9] The product according to [8] above, wherein the product is one selected from fragrance products, perfumery and cosmetic products, basic cosmetics, make-up cosmetics, hair cosmetics, sunburn cosmetics, medicated cosmetics, hair care products, soaps, body washes, bath preparations, fabric detergents, fabric softeners, detergents, kitchen detergents, bleaching agents, aerosols, air fresheners, repellents and miscellaneous goods.
[10] A method for enhancing or modulating the scent of a perfume composition, which comprises adding at least one compound according to any one of [1] to [5] above.

It should be noted that the compound of the present invention is a novel compound and its use is not limited to perfume materials.

### ADVANTAGEOUS EFFECTS OF INVENTION

The present invention aims to provide a novel compound. According to a preferred embodiment of the present invention, the compound of the present invention can be used as a novel perfume material having a musk-like scent.

### DESCRIPTION OF EMBODIMENTS

The present invention will be described in more detail below.

The compound of the present invention is characterized by being represented by the following general formula: 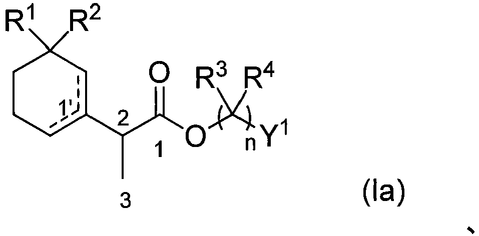 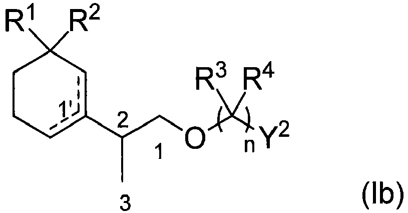 or 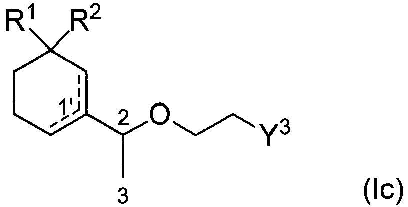 wherein
R¹ and R² are each independently a hydrogen atom or a methyl group,
n is 2 or 3,
two or more R³ and R⁴ are each independently a hydrogen atom or a methyl group,
Y¹ is -O-CO-Z, -O-CO-O-Z, -CO-Z, -CO-O-Z or -CH(Z¹)-CO-Z² , Y² is -O-CO-Z or -O-CO-O-Z, and Y³ is -O-CO-Z³, where Z is a linear or branched alkyl group containing 1 to 5 carbon atoms or a cycloalkyl group containing 3 to 5 carbon atoms, Z¹ is a hydrogen atom, a methyl group or an ethyl group, Z² is a linear or branched alkyl group containing 1 to 3 carbon atoms, and Z³ is a linear or branched alkyl group containing 1 to 5 carbon atoms or a cycloalkyl group containing 3 to 5 carbon atoms, and
the adjacent two ring-constituting double lines, each consisting of a solid line and a dotted line, are each independently a double bond or a single bond, except for the case where both are double bonds.

In the above formula, R¹ and R² are each independently a hydrogen atom or a methyl group, and R¹ and R² are both preferably methyl groups.
n is 2 or 3, and n is preferably 2.
Two or more R³ and R⁴ are each independently a hydrogen atom or methyl, and two or more R³ and R⁴ are all preferably hydrogen atoms.

Y¹ is -O-CO-Z, -O-CO-O-Z, -CO-Z, -CO-O-Z or -CH(Z¹)-CO-Z², preferably - O-CO-Z.
Y² is -O-CO-Z or -O-CO-O-Z, preferably -O-CO-Z.
Y³ is -O-CO-Z³.

Z is a linear or branched alkyl containing 1 to 5 carbon atoms or a cycloalkyl containing 3 to 5 carbon atoms, preferably a linear or branched alkyl containing 1 to 5 carbon atoms.
Z¹ is a hydrogen atom, methyl or ethyl, preferably a hydrogen atom.
Z² is a linear or branched alkyl containing 1 to 3 carbon atoms, preferably a methyl group.
Z³ is a linear or branched alkyl group containing 1 to 5 carbon atoms or a cycloalkyl group containing 3 to 5 carbon atoms, preferably a linear or branched alkyl group containing 1 to 3 carbon atoms or a cycloalkyl group containing 3 to 4 carbon atoms.

Herein, examples of a linear or branched alkyl group containing 1 to 5 carbon atoms include methyl, ethyl, n-propyl, isopropyl, n-butyl, isobutyl, sec-butyl, tert-butyl, n-amyl (n-pentyl), isoamyl (isopentyl), neopentyl and tert-amyl (tert-pentyl).

Examples of a linear or branched alkyl group containing 1 to 3 carbon atoms include methyl, ethyl, n-propyl and isopropyl.

Examples of a cycloalkyl group containing 3 to 5 carbon atoms include cyclopropyl, cyclobutyl and cyclopentyl.

The adjacent two ring-constituting double lines, each consisting of a solid line and a dotted line, are each independently a double bond or a single bond, except for the case where both are double bonds. In one embodiment of the present invention, the adjacent two ring-constituting double lines, each consisting of a solid line and a dotted line, are both preferably single bonds.

When the adjacent two ring-constituting double lines, each consisting of a solid line and a dotted line, are both single bonds, the compound of the present invention is not limited in any way as to its relative configuration, but the relative configuration at the 1'- and 2-positions is preferably (R^{∗},S^{∗}) or (R^{∗},R^{∗}) or a mixture of (R^{∗},S^{∗}) and (R^{∗},R^{∗}), and the relative configuration at the 1'- and 2-positions is more preferably (R^{∗},S^{∗}).

When the compound of the present invention is obtained as a mixture of the above structural isomers, the mixture may be used as such, but may also be separated by commonly known various techniques into the respective isomers for use alone. For example, when the compound of the present invention is used as a perfume material, if its structural isomers have greatly different aroma thresholds, it may be advantageous to selectively synthesize and use a compound having a lower aroma threshold.

In one embodiment of the present invention, a compound represented by the following general formula is preferred: 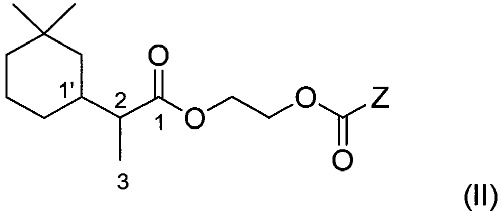 wherein Z is a linear or branched alkyl group containing 1 to 5 carbon atoms or a cycloalkyl group containing 3 to 5 carbon atoms.

Above all, particularly preferred is a compound represented by the following structural formula.

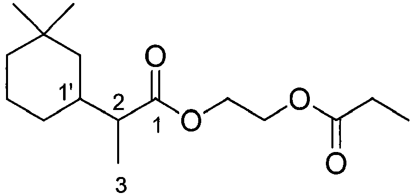

The compound represented by general formula (II) may be prepared, for example, by the process shown below.

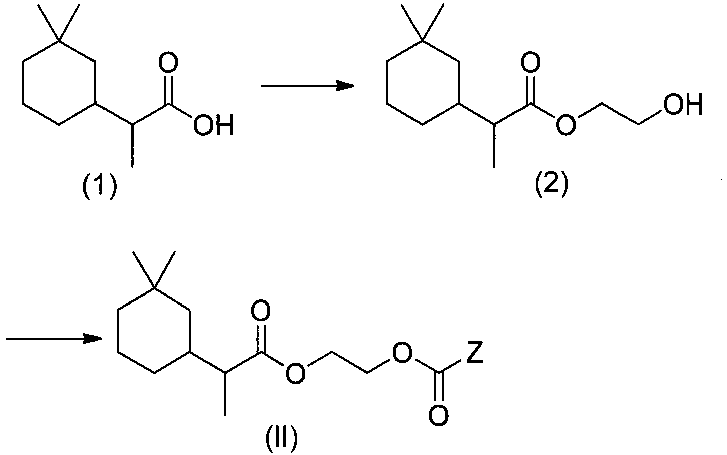

Namely, carboxylic acid (1) may be condensed with ethylene glycol to give alcohol (2), which may then be condensed with a carboxylic acid (ZCOOH) to thereby obtain the compound represented by general formula (II).

Any reaction solvent may be used for this purpose, and examples include ether-based solvents (e.g., tetrahydrofuran or diethyl ether); halogenated hydrocarbons (e.g., methylene chloride); halogenated aromatic hydrocarbons (e.g., o-dichlorobenzene); amides (e.g., N,N-dimethylformamide); sulfoxides (e.g., dimethyl sulfoxide); aromatic hydrocarbons (e.g., benzene, toluene); and aliphatic hydrocarbons (e.g., hexane).

In addition, 4,4-dimethylaminopyridine may preferably be used as a catalyst, while 1-ethyl-3-(3-dimethylaminopropyl)carbodiimide hydrochloride may preferably be used as a condensing agent.

The preparation process is not limited to this example, and commonly used condensation techniques may also be used.

The compound represented by general formula (II) may be obtained as a mixture of compounds represented by the following formulae (1a) and (1b).

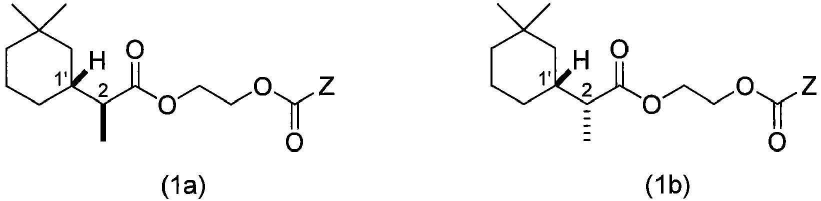

The compound of the present invention may be used as a mixture of the above structural isomers, but this mixture may also be separated by commonly known various techniques into the respective isomers for use alone. For example, when the compound of the present invention is used as a perfume material, if compound (1a) and compound (1b) have greatly different aroma thresholds, it may be advantageous to selectively synthesize and use a compound having a lower aroma threshold.

For example, when Z is an ethyl group, the aroma threshold of compound (1a) is about 100 times lower than that of compound (1b). In this case, it is preferable to selectively synthesize and use compound (1a).

It should be noted that a compound represented by the following general formula: 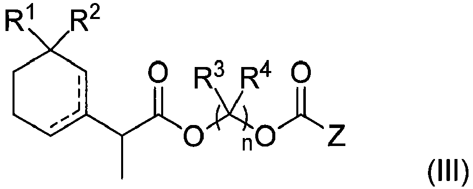 wherein the symbols in the formula are the same as those in general formulae (Ia) and (Ib),
may also be obtained in the same manner as described above when corresponding carboxylic acid and dihydric alcohol are used as starting compounds.

In another embodiment of the present invention, a compound represented by the following general formula is preferred: 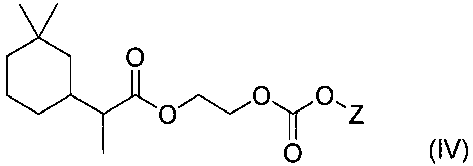 wherein Z is a linear or branched alkyl group containing 1 to 5 carbon atoms or a cycloalkyl group containing 3 to 5 carbon atoms.

The compound represented by general formula (IV) may be prepared, for example, by the process shown below.

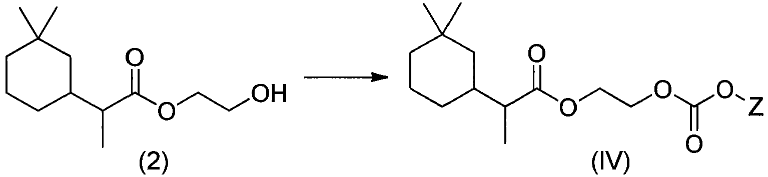

Namely, alcohol (2) may be condensed with an alkyl chloroformate or a cycloalkyl chloroformate to thereby obtain the compound represented by general formula (IV).

The reaction solvent used for this purpose may be the same as illustrated in the preparation process for the compound represented by general formula (II). In addition, triethylamine or the like may preferably be used as a base.

It should be noted that a compound represented by the following general formula: 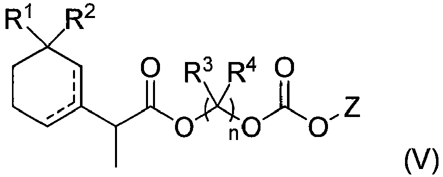 wherein the symbols in the formula are the same as those in general formulae (Ia) and (Ib),
may also be obtained in the same manner as described above when corresponding carboxylic acid and dihydric alcohol are used.

In yet another embodiment of the present invention, a compound represented by the following general formula is also particularly advantageous: 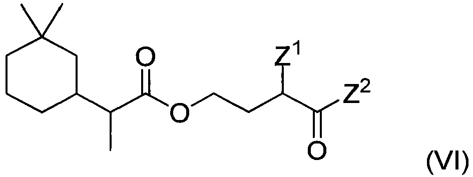 wherein Z¹ is a hydrogen atom, a methyl group or an ethyl group, and Z² is a linear or branched alkyl group containing 1 to 3 carbon atoms.

The compound represented by general formula (VI) may be obtained, for example, by the process shown below: 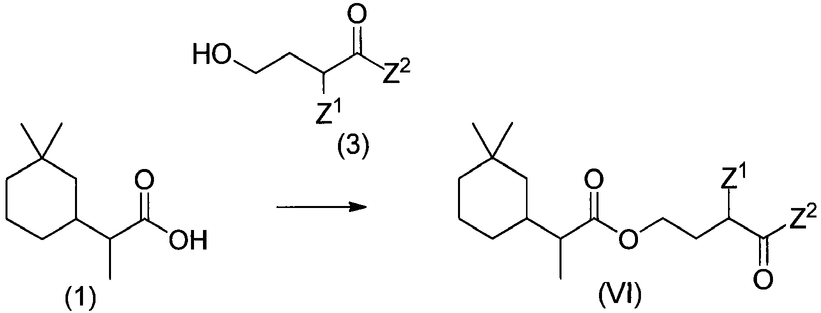 wherein the symbols in the formulae are the same as those in general formulae (Ia) and (Ib).

Namely, carboxylic acid (1) may be condensed with a compound represented by general formula (3) to thereby obtain the compound represented by general formula (VI).

The reaction solvent and reagents used for this purpose may be the same as illustrated in the preparation process for the compound represented by general formula (II).

It should be noted that a compound represented by the following general formula: 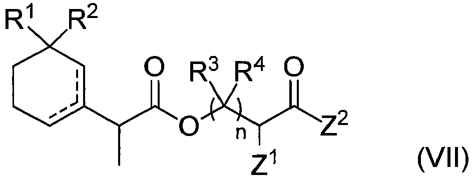 wherein the symbols in the formula are the same as those in general formulae (Ia) and (Ib),
may also be obtained in the same manner as described above when corresponding carboxylic acid and alcohol are condensed with each other.

In a further embodiment of the present invention, a compound represented by the following general formula is also preferred: 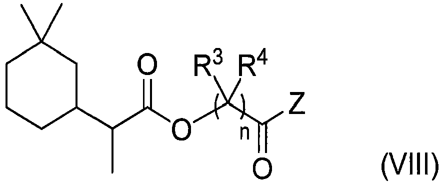 wherein n is 2 or 3, two or more R³ and R⁴ are each independently a hydrogen atom or a methyl group, and Z is a linear or branched alkyl group containing 1 to 5 carbon atoms or a cycloalkyl group containing 3 to 5 carbon atoms.

The compound represented by general formula (VIII) may be obtained, for example, by the process shown below: 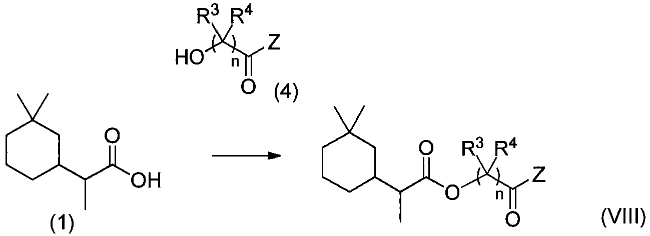 wherein the symbols in the formulae are the same as those in general formulae (Ia) and (Ib).

Namely, carboxylic acid (1) may be condensed with a compound represented by general formula (4) to thereby obtain the compound represented by general formula (VIII).

The reaction solvent and reagents used for this purpose may be the same as illustrated in the preparation process for the compound represented by general formula (II).

It should be noted that a compound represented by the following general formula: 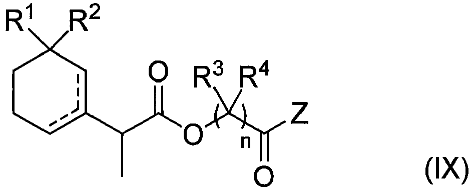 wherein the symbols in the formula are the same as those in general formulae (Ia) and (Ib),
may also be obtained in the same manner as described above when corresponding carboxylic acid and alcohol are condensed with each other.

In a further embodiment of the present invention, a compound represented by the following general formula is also preferred: 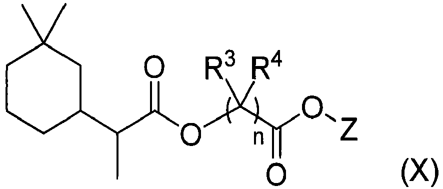 wherein n is 2 or 3, two or more R³ and R⁴ are each independently a hydrogen atom or a methyl group, and Z is a linear or branched alkyl group containing 1 to 5 carbon atoms or a cycloalkyl group containing 3 to 5 carbon atoms.

The compound represented by general formula (X) may be obtained, for example, by the process shown below: 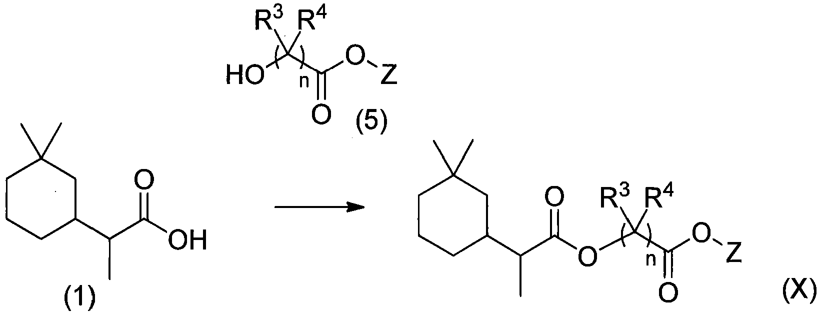 wherein the symbols in the formulae are the same as those in general formulae (Ia) and (Ib).

Namely, carboxylic acid (1) may be condensed with a compound represented by general formula (5) to thereby obtain the compound represented by general formula (X).

The reaction solvent and reagents used for this purpose may be the same as illustrated in the preparation process for the compound represented by general formula (II).

It should be noted that a compound represented by the following general formula: 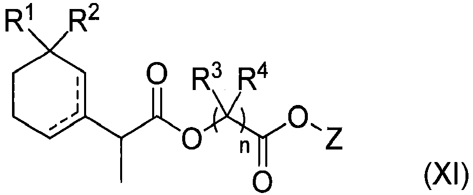 wherein the symbols in the formula are the same as those in general formulae (Ia) and (Ib),
may also be obtained in the same manner as described above when corresponding carboxylic acid and alcohol are condensed with each other as starting compounds.

In a further embodiment of the present invention, a compound represented by the following general formula is also preferred: 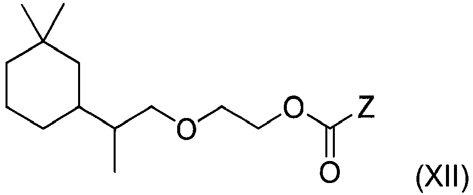 wherein Z is a linear or branched alkyl group containing 1 to 5 carbon atoms or a cycloalkyl group containing 3 to 5 carbon atoms.

The compound represented by general formula (XII) may be obtained, for example, by the process shown below.

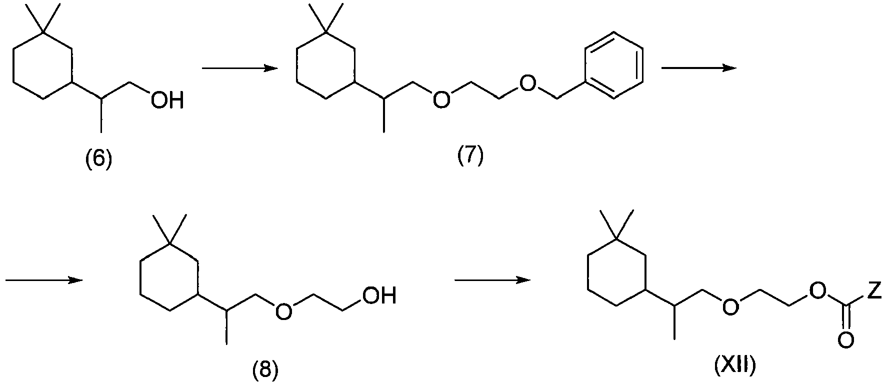

Namely, a hydroxyethyl group protected with a benzyl group may be elongated from alcohol (6) through Williamson ether synthesis to obtain compound (7), followed by deprotection with a palladium catalyst to obtain compound (8). Then, compound (8) may be condensed with a carboxylic acid (ZCOOH) to thereby obtain the compound represented by general formula (XII).

In the condensation reaction, the reaction solvent and reagents used for this purpose may be the same as illustrated in the preparation process for the compound represented by general formula (II). Likewise, the Williamson ether synthesis may be conducted as appropriate by those skilled in the art while referring to Examples 13 and 14.

It should be noted that a compound represented by the following general formula: 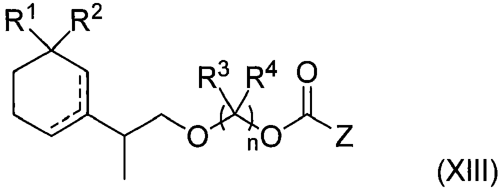 wherein the symbols in the formula are the same as those in general formulae (Ia) and (Ib),
may also be obtained in the same manner as described above when a corresponding alcohol is used as a starting compound.

Moreover, a corresponding alcohol such as the compound represented by general formula (8) may be used as an intermediate, and this compound may be condensed with an alkyl chloroformate or a cycloalkyl chloroformate to thereby obtain a compound represented by the following general formula: 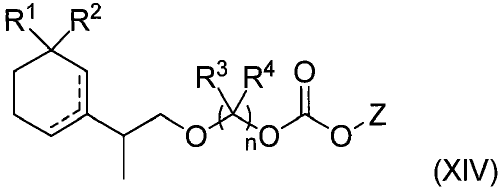 wherein the symbols in the formula are the same as those in general formulae (Ia) and (Ib).

It should be noted that the reaction solvent used for this purpose may be the same as illustrated in the preparation process for the compound represented by general formula (II). In addition, triethylamine or the like may preferably be used as a base.

In a further embodiment of the present invention, a compound represented by the following general formula is also preferred: 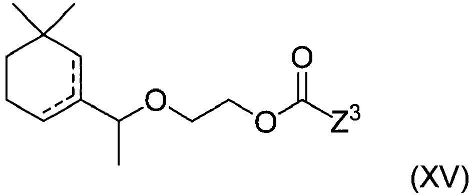 wherein Z³ is a linear or branched alkyl group containing 1 to 5 carbon atoms or a cycloalkyl group containing 3 to 5 carbon atoms.

The compound represented by general formula (XV) may be obtained, for example, by the process shown below.

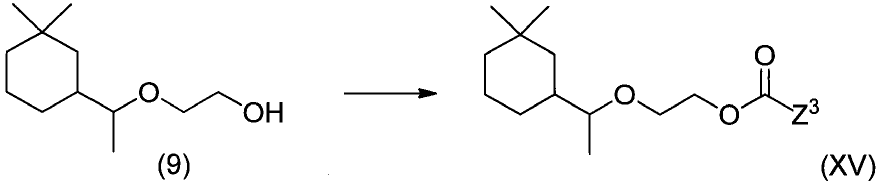

Namely, alcohol (9) may be condensed with a carboxylic acid (Z³COOH) to thereby obtain the compound represented by general formula (XV).

In the condensation reaction, the reaction solvent and reagents used for this purpose may be the same as illustrated in the preparation process for the compound represented by general formula (II).

It should be noted that a compound represented by the following general formula: 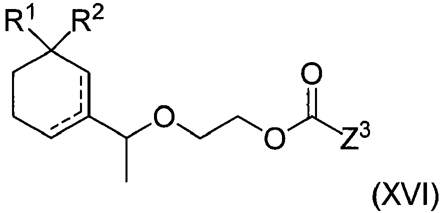 wherein the symbols in the formula are the same as those in general formula (Ic),
may also be obtained in the same manner as described above when a corresponding alcohol is used as a starting compound.

The thus obtained compounds may be isolated and purified, as needed. Techniques for isolation and purification include, for example, column chromatography, vacuum distillation, crystallization and so on, which may be used alone or in combination.

According to a preferred embodiment of the present invention, the compound of the present invention is useful as a perfume material which imparts a musk-like scent. According to another preferred embodiment of the present invention, the scent of a perfume composition may be enhanced or modulated upon addition of at least one compound of the present invention.

The perfume composition of the present invention is characterized by containing at least one of the above compounds according to the present invention.

The compounds according to the present invention may be contained alone, or two or more of them may be contained in combination. Moreover, the compound of the present invention may be contained as a mixture of structural isomers.

The content of the compound of the present invention in the perfume composition of the present invention may be determined as appropriate depending on the intended use and/or purpose and is not limited in any way. In general, it is preferably in the range of 0.0001% to 80% by mass, more preferably 0.001% to 60% by weight, particularly preferably 0.01% to 40% by weight, based on the total weight of the perfume composition.

Moreover, the perfume composition of the present invention may comprise a compounded perfume which is commonly used, in addition to the compound of the present invention.

According to a preferred embodiment of the present invention, the perfume composition of the present invention enables the provision of a fresh and highly palatable scent.

The perfume composition of the present invention may be incorporated as an aroma component into fragrance products, perfumery and cosmetic products, basic cosmetics, make-up cosmetics, hair cosmetics, sunburn cosmetics, medicated cosmetics, hair care products, soaps, body washes, bath preparations, fabric detergents, fabric softeners, detergents, kitchen detergents, bleaching agents, aerosols, air fresheners, repellents and miscellaneous goods, etc.

For example, the perfume composition of the present invention may be incorporated in an amount commonly used in the art into the following:
various hair cosmetic bases such as shampoos, conditioners, perfumed water, colognes, hair tonics, hair creams, pomades and others;
various sanitary detergents such as soaps, dishwashing detergents, laundry detergents, softeners, antiseptic detergents, deodorizing detergents, indoor air fresheners, antiseptics, insecticides, bleaching agents and others; and
dentifrices, mouthwashes, toilet paper, and perfuming agents which facilitate the intake of medicaments, etc.

When a product is scented with the perfume composition of the present invention, the unique scent of the perfume composition of the present invention can be imparted to the product, so that the commercial value of the product can be increased.

### EXAMPLES

The present invention will be further described in more detail by way of the following illustrative examples, although the present invention is not limited only to these examples. It should be noted that the instruments shown below were used for various measurements in this Example section.
NMR instrument (¹H-NMR, ¹³C-NMR): AVANCE III 500 (Bruker)
Internal standard: TMS or CDCl₃
Gas chromatograph/mass spectrometer (GC/MS): GCMS-QP2010 Ultra (Shimadzu Corporation, Japan)
Column used: Rxi-5ms (RESTEK) (30.0 m length × 0.25 mm inner diameter, 0.25 µm film thickness, Shimadzu GLC Ltd., Japan)
Gas chromatography (GC, chemical purity): 7890A (Agilent Technologies)
Column used: InertCap 1 (20 m length × 0.18 mm inner diameter, 0.18 µm film thickness, GL Sciences Inc., Japan)

### [Example 1 (reference example)] 2-Hydroxyethyl 2-(3,3 -dimethylcyclohexyl)propanonate

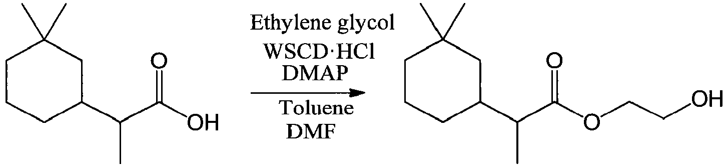

A mixed solution of toluene (10 ml), dimethylformamide (DMF, 5 ml) and 2-(3,3-dimethylcyclohexyl)propanoic acid (22.0 g, 119 mmol) was cooled to 0°C under stirring conditions, to which ethylene glycol (23.4 ml), 4,4-dimethylaminopyridine (725 mg, 5.93 mmol) and 1-ethyl-3-(3-dimethylaminopropyl)carbodiimide hydrochloride (hereinafter referred to as WSCD·HCl) (25.1 g, 130 mmol) were then added sequentially while keeping the interior of the system at 5°C or less. This mixture was warmed and stirred at 15°C for 1 hour. The mixture was then cooled to 0°C and 1 M hydrochloric acid (5 ml) was added thereto, followed by vigorous stirring. This mixture was partitioned to obtain an organic layer, which was then washed once with 20% aqueous sodium bicarbonate and three times with 5% aqueous sodium chloride. After the solvent was distilled off with a rotary evaporator, the residue was distilled at 80 Pa/104°C to obtain 2-hydroxyethyl 2-(3,3-dimethylcyclohexyl)propanonate (7.32 g, 32.1 mmol, yield: 27%). Stereoisomers were purified and separated by recycling column chromatography (silica gel, hexane:ethyl acetate = 4:1).

### Isomer of the relative configuration (1'R^{∗},2S^{∗})

¹H-NMR (400 MHz, CDCl₃): 0.77(d/q, J = 3.90 Hz, 13.0 Hz, 1H), 0.89(d, J = 3.10 Hz, 6H), 0.91(t, J = 12.5 Hz, 1H), 1.49(t/d, J = 13.4, 4.10 Hz Hz, 1H), 1.3(d, J = 7.00 Hz, 3H), 1.24~1.29(m, 1H), 1.32~1.37(m, 1H), 1.42(q/t, J = 13.4, 3.50 Hz, 1H), 1.55~1.65(m, 1H), 1.69~1.78(m, 2H), 1.95(s, 1H), 2.23(quint, J = 7.10 Hz, 1H), 3.81~3.84(m, 2H), 4.22(m, 2H)

¹³C-NMR (125 Hz, CDCl₃): 176.88(s), 65.75(t), 61.47(t), 45.63(d), 44.19(t), 38.98(t), 36.53(d), 33.44(q), 30.83(s), 29.44(t), 24.56(q), 22.16(t), 14.11(q)

GC/MS (m/e): 229(1), 213(2), 185(2), 167(5), 153(3), 139(6), 123(10), 118(100), 100(33), 95(28), 83(15), 69(33), 56(30), 41(22), 29(7)

### Isomer of the relative configuration (1'R^{∗},2R^{∗})

¹H-NMR (¹H, 400 MHz, CDCl₃): 0.81(t, J = 12.7 Hz, 1H), 0.9(d, J = 9.90 Hz, 6H), 0.87~0.93(m, 1H), 1.05(d/t, J = 2.10 Hz, 6.60 Hz, 1H), 1.12(d, 7.00 Hz, 3H), 1.32~1.38(m, 1H), 1.36~1.48(m, 2H), 1.52~1.58(m, 1H), 1.58~1.63(m, 1H), 1.73(m, 1H), 1.97~2.04(s, 1H), 2.24(quint. J = 7.15 Hz, 1H), 3.83(q, J = 4.75 Hz, 2H), 4.22(m, 2H)

¹³C-NMR (125 Hz, CDCl₃): 177.01(s), 65.75(t), 61.43(t), 45.53(d), 42.69(t), 38.96(t), 36.59(d), 33.49(q), 30.98(s), 30.78(t), 24.64(q), 22.19(t), 13.93(q)

GC/MS (m/e): 229(1), 213(2), 185(3), 167(7), 153(4), 139(7), 123(11), 118(100), 95(27), 83(15), 69(35), 56(32), 45(8), 41(23), 29(6)

### [Example 2]

### 2-(Propionyloxy)ethyl 2-(3,3 -dimethylcyclohexyl)propanonate

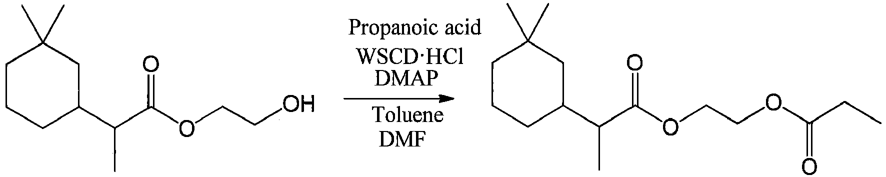

A mixed solution of toluene (8 ml), dimethylformamide (DMF, 3 ml) and 2-hydroxyethyl 2-(3,3-dimethylcyclohexyl)propanonate (7.32 g, 32.1 mmol) was cooled to 0°C under stirring conditions, to which propanoic acid (also referred to as propionic acid) (2.6 ml), 4,4-dimethylaminopyridine (194 mg, 1.58 mmol) and WSCD·HCl (6.76 g, 35.2 mmol) were then added sequentially while keeping the interior of the system at 5°C or less. This mixture was warmed and stirred at 15°C for 1 hour. The mixture was then cooled to 0°C and 1 M hydrochloric acid (5 ml) was added thereto, followed by vigorous stirring. This mixture was partitioned to obtain an organic layer, which was then washed once with 20% aqueous sodium bicarbonate and three times with 5% aqueous sodium chloride. After the solvent was distilled off with a rotary evaporator, the residue was distilled at 37 Pa/95.8°C to obtain 2-(propionyloxy)ethyl 2-(3,3-dimethylcyclohexyl)propanonate (6.93 g, 24.4 mmol, yield: 76%). Stereoisomers were purified and separated by recycling column chromatography (silica gel, hexane:ethyl acetate = 9:1).

### Isomer of the relative configuration (1'R^{∗},2S^{∗})

¹H-NMR (400 MHz, CDCl₃): 0.79(q/d, J = 13.0, 3.95 Hz, 1H), 0.88(s, 3H), 0.89(s, 3H), 0.86~0.93(t, J = 12.6 Hz, 1H), 1.04(t/d, 13.4, 4.20 Hz, 1H), 1.11(d, J = 7.00 Hz, 3H), 1.15(t, J = 7.60 Hz, 3H), 1.26(m, 1H), 1.34(m, 1H), 1.42(q/t, J = 13.5, 3.60 Hz, 1H), 1.54~1.61(m, 1H), 1.67~1.77(m, 2H), 2.21(quint. J = 7.1 Hz, 1H), 2.35(q, 7.60 Hz, 2H), 4.30(s, 4H)

¹³C-NMR (125 Hz, CDCl₃): 176.20(s), 174.21(s), 62.16(t), 61.74(t), 45.57(d), 44.11(t), 39.00(t), 36.44(d), 33.45(q), 30.83(s), 29.40(t), 27.42(t), 24.54(q), 22.18(t), 14.01(q), 9.03(q)

GC/MS (m/e): 211(1), 195(1), 174(12), 167(3), 139(7), 111(2), 101(100), 100(52), 83(10), 79(3), 69(18), 57(30), 41(12), 39(3), 29(15)

### Isomer of the relative configuration (1'R^{∗},2R^{∗})

¹H-NMR (400 MHz, CDCl₃): 0.81(t, J = 12.6 Hz, 1H), 0.83~0.92(m, 1H), 0.89(s, 3H), 0.91(s, 3H), 1.04(t/d, J = 9.05 Hz, 4.20 Hz, 1H), 1.10(d, J = 7.00 Hz, 3H), 1.15(t, J = 7.55 Hz, 3H), 1.31~1.40(m, 2H), 1.40~1.47(t/t, J = 13.3, 3.65 Hz, 1H), 1.51~1.57(m, 1H), 1.59~1.63(m, 1H), 1.72(m, 1H), 2.22(quint. J = 7.10 Hz, 1H), 2.35(q, J = 7.60 Hz, 2H), 4.23~4.34(m, 4H)

¹³C-NMR (125 Hz, CDCl₃): 176.30(s), 174.20(s), 62.15(t), 61.73(t), 45.52(d), 42.71(t), 38.99(t), 36.53(d), 33.51(q), 30.91(s), 30.80(t), 27.41(t), 24.64(q), 22.21(t), 13.92(q), 9.02(q)

GC/MS (m/e): 210(1), 195(1), 174(12), 167(3), 139(7), 111(3), 101(100), 100(55), 83(12), 79(3), 69(20), 57(32), 43(4), 41(12), 29(15)

### [Example 3]

### 2-(Propionyloxy)ethyl 2-cyclohexylpropanonate

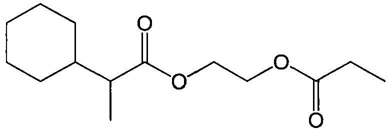

The same procedures as shown in Examples 1 and 2 were repeated to synthesize the titled compound, except for starting from 2-cyclohexylpropionic acid instead of 2-(3,3-dimethylcyclohexyl)propanoic acid.

¹H-NMR (400 MHz, CDCl₃): 0.85~1.10(m, 2H), 1.11(d, J = 7.00 Hz, 3H), 1.15(t, 7.55 Hz, 4H), 1.18~1.31(m, 2H), 1.54(m, 1H), 1.63(m, 2H), 1.68~1.77(m, 3H), 2.27 (quint. J = 7.10 Hz, 1H), 2.35(q, J = 8.15 Hz, 2H), 4.45(m, 4H)

¹³C-NMR (125 Hz, CDCl₃): 176.24(s), 174.18(s), 62.10(t), 61.71(t), 45.36(d), 40.74(d), 31.06(t), 29.61(t), 27.41(t), 26.30(t), 26.28(t), 26.24(t), 13.95(q), 9.02(q)

GC/MS (m/e): 257(1), 227(1), 183(1), 174(10), 157(1), 139(12), 126(3), 111(22), 101(100), 95(1), 81(5), 69(20), 57(42), 41(15), 29(18)

### [Example 4]

### 2-((2-(3,3-Dimethylcyclohexyl)propanoyl)oxy)ethyl cyclopropanecarboxylate

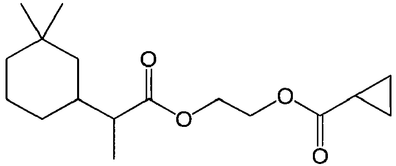

The same procedures as shown in Example 2 were repeated to synthesize the titled compound, except that cyclopropanoic acid was used instead of propionic acid.

### Isomer of the relative configuration (1'R^{∗},2S^{∗})

¹H-NMR (400 MHz, CDCl₃): 0.81(q/d, 12.8, 3.85 Hz, 1H), 0.85~0.94(m, 3H), 0.89(s, 6H), 0.99~1.03(m, 2H), 1.04(t/d, 13.3, 3.90 Hz, 1H), 1.12(d, J = 7.00 Hz, 3H), 1.26(d/q, J = 12.8, 2.5 Hz, 1H), 1.34(m, 1H), 1.42(q/t, J = 13.4, 6.15 Hz, 1H), 1.54~1.61(m, 1H), 1.63(t/t, J = 8.05, 4.60 Hz, 1H), 1.69~1.78(m, 2H), 2.22(quint. J = 6.95 Hz, 1H), 4.28(s, 4H)

¹³C-NMR (125 Hz, CDCl₃): 176.19(s), 174.63(s), 62.29(t), 61.77(t), 45.59(d), 44.11(t), 39.01(t), 36.45(d), 33.45(q), 30.84(s), 29.42(t), 24.55(q), 22.19(t), 14.01(d), 12.77(d), 8.59(t), 8.59(t)

GC/MS (m/e): 211(1), 195(1), 186(18), 156(2), 141(2), 139(8), 123(5), 113(100), 109(8), 100(88), 83(17), 69(88), 55(22), 41(38), 29(6)

### Isomer of the relative configuration (1'R^{∗},2R^{∗})

¹H-NMR (400 MHz, CDCl₃): 0.816(t, J = 12.6 Hz, 1H), 0.85~0.90(m, 1H), 0.89(s, 6H), 0.91(s, 3H), 1.10(d/t, J = 7.50, 3.65 Hz, 2H), 1.06(d/d, J = 13.0, 3.90 Hz, 1H), 1.10(d, J = 7.00 Hz, 3H), 1.31~1.47(m, 4H), 1.51~1.57(m, 1H), 1.62(t/t, J = 8.00, 4.70 Hz, 2H), 1.68~1.77(m, 1H), 2.23(quint. 7.10 Hz, 1H), 4.23~4.34(m, 4H)

¹³C-NMR (125 Hz, CDCl₃): 176.29(s), 174.63(s), 62.27(t), 61.76(t), 45.53(d), 42.72(t), 39.00(t), 36.55(d), 33.52(q), 30.92(t), 30.81(t), 24.65(q), 22.23(t), 13.92(d), 12.75(d), 8.57(t), 8.57(t)

GC/MS (m/e): 211(1), 195(1), 186(18), 156(2), 141(1), 139(8), 123(6), 113(100), 101(6), 100(93), 83(17), 69(95), 55(22), 41(39), 29(6)

### [Example 5]

### 2-Acetoxyethyl 2-(3,3 -dimethylcyclohexyl)propanonate

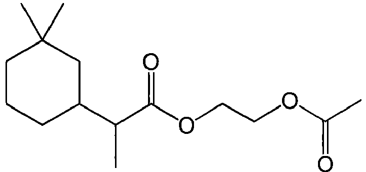

The same procedures as shown in Example 2 were repeated to synthesize the titled compound, except that acetic acid was used instead of propionic acid. GC/MS (m/e):
Isomer 1: 211(1), 185(1), 167(3), 160(15), 139(5), 109(3), 100(40), 87(100), 79(2), 69(22), 55(15), 43(30), 41(14), 29(3)
Isomer 2: 211(1), 185(1), 167(3), 160(15), 139(7), 109(3), 100(40), 87(100), 79(3), 69(23), 55(16), 43(32), 41(15), 29(3)

### [Example 6]

### 2-((2-(3,3-Dimethylcyclohexyl)propanoyl)oxy)ethyl cyclobutanecarboxylate

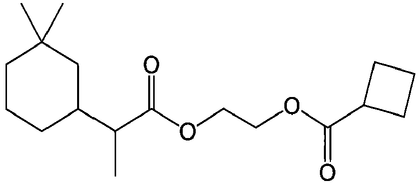

The same procedures as shown in Example 2 were repeated to synthesize the titled compound, except that cyclobutanoic acid was used instead of propionic acid.

### Isomer of the relative configuration (1'R^{∗},2S^{∗})

¹H-NMR (400 MHz, CDCl₃): 0.79(q/d, J = 13.1, 4.05 Hz, 1H), 0.88(s, 3H), 0.89(s, 3H), 0.89(t, J = 12.5 Hz, 1H), 1.04(t/d, J = 13.1, 4.10 Hz, 1H), 1.11(d, J = 7.00 Hz, 3H), 1.25(d/q, 13.0, 2.45 Hz, 1H), 1.31~1.37(m, 1H), 1.41(q/t, 13.4, 3.55 Hz, 1H), 1.53~1.61(m, 1H), 1.69~1.73(m, 2H), 1.86~2.03(m, 2H), 2.16~2.24(m, 3H), 2.25~2.34(m, 2H), 3.15(quint, J =8.55 Hz, 1H), 4.27(s, 4H)

¹³C-NMR (125 Hz, CDCl₃): 176.17(s), 175.21(s), 62.12(t), 61.79(t), 45.55(d), 44.10(t), 39.01(t), 37.94(d), 36.41(d), 33.44(q), 30.83(s), 29.38(t), 25.22(t), 25.22(t), 24.54(q), 22.18(t), 18.40(t), 13.97(q)

GC/MS (m/e): 255(1), 211(3), 200(12), 182(1), 167(5), 145(2), 139(10), 127(95), 109(8), 100(100), 83(50), 69(28), 57(12), 55(75), 41(20), 29(12)

### Isomer of the relative configuration (1'R^{∗},2R^{∗})

¹H-NMR (400 MHz, CDCl₃): 0.80(t, J = 12.9 Hz, 1H), 0.83~0.89(m, 1H), 0.88(s, 3H), 0.91(s, 3H), 1.05(t/d, 13.2, 4.05 Hz, 1H), 1.1(d, 7.00 Hz, 3H), 1.31~1.47(m, 3H), 1.51~1.54(m, 1H), 1.59(m, 1H), 1.71(m, 1H), 1.86~2.03(m, 2H), 2.16~2.24(m, 3H), 2.29(m, 2H), 3.15(quint/d, J = 8.60, 0.85 Hz, 1H), 4.24~4.33(m, 4H)

¹³C-NMR (125 Hz, CDCl₃): 176.29(s), 175.19(s), 62.10(t), 61.78(t), 45.57(d), 42.73(t), 38.99(t), 37.94(d), 36.53(d), 33.51(q), 30.94(t), 30.79(s), 25.22(t), 25.21(t), 24.64(q) 22.21(t), 18.4(t), 13.97(q)

GC/MS (m/e): 255(1), 211(3), 200(12), 182(1), 167(5), 145(2), 139(10), 127(95), 109(8), 100(100), 83(50), 69(28), 57(12), 55(75), 41(20), 29(12)

### [Example 7]

### 2-((2-(3,3-Dimethylcyclohexyl)propanoyl)oxy)ethyl isobutyrate

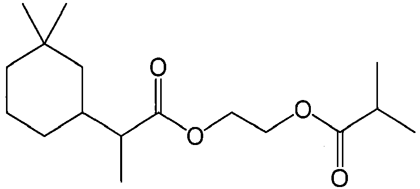

The same procedures as shown in Example 2 were repeated to synthesize the titled compound, except that isobutyric acid was used instead of propionic acid.

¹H-NMR (400 MHz, CDCl₃): 0.77(t/d, J = 12.8, 4.00 Hz, 1H), 0.86(s, 6H), 0.97~1.08(m, 1H), 1.02~1.10(m, 6H), 1.22~1.28(d, J = 7.20 Hz, 3H), 1.29~1.38(m, 1H), 1.28~1.49(m, 3H), 1.49~1.52(m, 2H), 1.62~1.77(m, 1H), 2.18(quint. 1H), 2.54(heptet. 1H), 4.25(s, 4H) (diastereomer 1:1 mixture)

GC/MS (m/e):
Isomer 1: 255(1), 225(1), 211(1), 189(1), 188(12), 167(3), 151(1), 139(3), 123(2), 115(100), 100(75), 83(8), 71(30), 57(8), 55(20), 43(40), 29(5)
Isomer 2: 255(1), 225(1), 211(1), 195(1), 188(12), 167(3), 151(1), 139(3), 123(2), 115(100), 100(75), 83(8), 71(30), 57(8), 55(20), 43(40), 29(5)

### [Example 8]

### 2-(Propionyloxy)ethyl 2-(3-methylcyclohex-1-en-1-yl)propanonate

### 2-(Propionyloxy)ethyl 2-(5-methylcyclohex-1-en-1-yl)propanonate

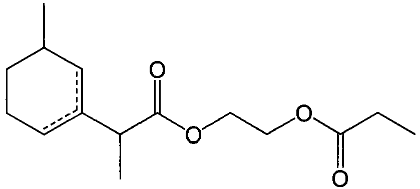

The same procedures as shown in Examples 1 and 2 were repeated to synthesize the titled compounds, except for starting from 3-methylcyclohexen-1-ylpropane (as a mixture of positional isomers).

GC/MS (m/e):
Isomer 1: 211(1), 195(1), 166(1), 150(1), 135(1), 122(100), 101(50), 95(38), 79(15), 67(12), 57(45), 41(11), 39(3) 29(25)
Isomer 2: 211(1), 194(1), 166(1), 150(1), 135(1), 122(100), 107(20), 101(60), 91(8), 81(22), 67(12), 57(50), 43(3), 41(7), 29(25)
Isomer 3: 211(1), 194(1), 167(1), 150(1), 135(1), 122(100), 101(65), 93(45), 81(25), 67(13), 57(58), 43(6), 41(18), 29(28)

### [Example 9]

### 2-(Propionyloxy)ethyl 2-(3-methylcyclohexyl)propanonate

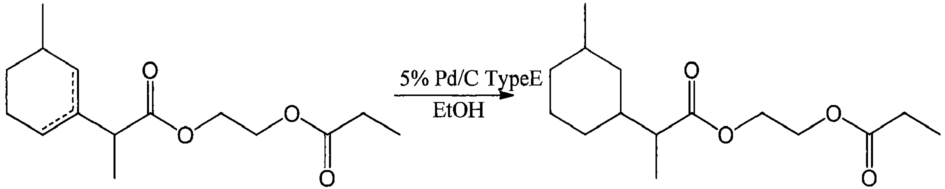

Under a nitrogen atmosphere, to a mixed solution prepared from the isomer mixture of 2-(propionyloxy)ethyl 2-(3-methylcyclohex-1-en-1-yl)propanonate and 2-(propionyloxy)ethyl 2-(5-methylcyclohex-1-en-1-yl)propanonate obtained in Example 8 (201 mg, 0.75 mmol) and ethanol (1.5 ml), 5% Pd/C Type E (2.1 mg, 1 wt%) was added under nitrogen, and the reaction vessel was purged with hydrogen, followed by stirring at 50°C for 6 hours. Then, the reaction mixture was filtered through celite, and the solvent was distilled off with a rotary evaporator to obtain 2-(propionyloxy)ethyl 2-(3-methylcyclohexyl)propanonate (188 mg, 070 mmol, yield: 93%) as a crude product.

GC/MS (m/e):
Isomer 1: 241(1), 197(1), 181(1), 174(11), 152(6), 109(1), 101(100), 83(5), 69(18), 67(5), 57(45), 41(15), 29(20)
Isomer 2: 241(1), 197(1), 181(1), 174(11), 152(5), 109(1), 101(100), 83(5), 69(19), 67(5), 57(45), 41(15), 29(18)
Isomer 3: 197(1), 181(1), 174(10), 153(5), 125(12), 119(3), 101(100), 100(60), 79(3), 69(20), 67(5), 57(45), 41(15), 29(21)
Isomer 4: 197(1), 181(1), 174(8), 153(5), 125(7), 119(3), 101(100), 100(61), 79(3), 69(18), 57(49), 41(15), 29(21)

### [Example 10]

### 2-((Ethoxycarbonyl)oxy)ethyl 2-(3,3 -dimethylcyclohexyl)propanonate

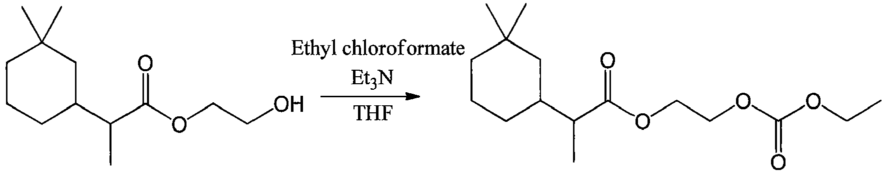

A mixture of 2-hydroxyethyl 2-(3,3-dimethylcyclohexyl)propanonate (338 mg, 1.70 mmol), triethylamine (1 ml) and tetrahydrofuran (THF, 3 ml) was cooled to 0°C, and ethyl chloroformate (162 µl, 1.70 mmol) was added dropwise thereto while keeping the mixture at 5°C or less. Then, the mixture was stirred at 15°C for 30 minutes. After addition of 20% aqueous ammonium chloride and toluene, the mixture was vigorously stirred and the organic layer was then separated. The organic layer was washed twice with water (5 ml). After the solvent was distilled off with a rotary evaporator, the residue was purified by column chromatography (silica gel, hexane:ethyl acetate = 9:1) to obtain 2-((ethoxycarbonyl)oxy)ethyl 2-(3,3-dimethylcyclohexyl)-propanonate (80.3 mg, 0.27 mmol, yield: 16%).

¹H-NMR (400 MHz, CDCl₃): 0.76~0.90(m, 1H), 0.88(s, 3H), 0.89(s, 3H), 0.88~0.93(m, 1H), 1.04(t/d, J = 13.2, 4.20 Hz, 1H), 1.1(t, 7.00 Hz, 3H), 1.23~1.34(m, 4H), 1.32~1.47(m, 2H), 1.51~1.77(m, 3H), 2.18~2.26(m, 1H), 4.21(q, 7.10 Hz, 2H), 4.25~4.35(m, 4H) (diastereomer 1:1 mixture)

GC/MS (m/e):
Isomer 1: 210(1), 190(5), 167(3), 146(1), 139(7), 123(3), 117(12), 101(6), 100(100), 89(11), 79(1), 69(18), 55(13), 45(12), 41(11), 29(10)
Isomer 2: 210(1), 190(5), 167(3), 146(1), 139(8), 117(11), 101(6), 100(100), 89(12), 79(1), 69(18), 55(15), 41(12), 39(1), 29(10)

### [Example 11]

### 1-((2-(3,3-Dimethylcyclohexyl)propanoyl)oxy)propan-2-yl cyclopropanecarboxylate

### 2-((2-(3,3-Dimethylcyclohexyl)propanoyl)oxy)propyl cyclopropanecarboxylate

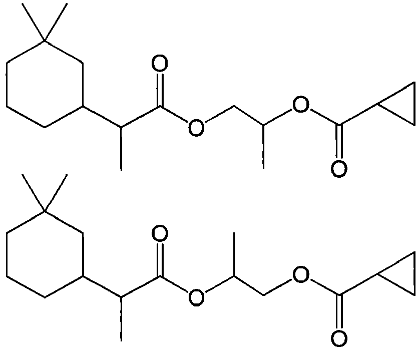

The same procedures as shown in Example 1 were repeated to synthesize corresponding alcohol forms, except that 1,2-propanediol was used instead of ethylene glycol, and the same procedures as shown in Example 2 were then repeated to synthesize the titled compounds, except that cyclopropanoic acid was used instead of propionic acid.

GC/MS:
Isomer 1: 224(1), 201(1), 200(12), 181(1), 167(5), 145(2), 139(15), 127(60), 114(84), 95(18), 83(18), 69(100), 55(18), 41(35), 29(3)
Isomer 2: 224(1), 209(1), 200(12), 181(1), 167(6), 145(2), 139(10), 127(65), 114(75), 95(18), 83(17), 69(100), 55(20), 41(35), 29(5)
Isomer 3: 224(1), 209(1), 200(15), 181(1), 167(5), 145(2), 139(10), 127(60), 114(85), 95(20), 83(20), 69(100), 55(20), 41(35), 29(5)
Isomer 4: 200(15), 156(5), 139(10), 127(60), 114(85), 109(5), 95(6), 83(15), 81(10), 69(100), 55(22), 43(7), 41(35), 29(5)

### [Example 12]

### 3-(Propionyloxy)propyl 2-(3,3 -dimethylcyclohexyl)propanonate

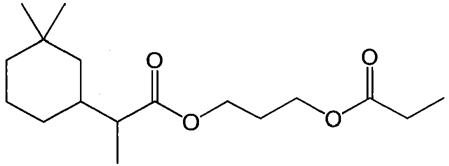

The same procedures as shown in Example 1 were repeated to synthesize a corresponding alcohol form, except that 1,3-propanediol was used instead of ethylene glycol, and the same procedures as shown in Example 2 were then repeated to synthesize the titled compound.

¹H-NMR (400 MHz, CDCl₃): 0.73~0.85(m, 1H), 0.88(s, 3H), 0.89~0.94(m, 4H), 1.04(t/d, J = 13.4, 4.15, 1H), 1.1(t, J = 7.10 Hz, 3H), 1.14(t, J = 7.55 Hz, 3H), 1.24~1.47(m, 3H), 1.51~1.79(m, 3H), 1.98(quint. J = 6.35 Hz, 2H), 2.18(t/d, J = 14.3, 7.10 Hz, 1H), 2.34(q, J = 7.60 Hz, 2H), 4.10~4.21(m, 4H) (diastereomer 1:1 mixture)

GC/MS (m/e):
Isomer 1: 241(1), 224(1), 209(1), 189(1), 188(12), 167(3), 151(1), 139(8), 123(4), 115(100), 95(15), 83(12), 69(18), 57(33), 41(12), 29(10)
Isomer 2: 241(1), 224(1), 209(1), 189(1), 188(12), 167(3), 151(1), 139(8), 123(4), 115(100), 95(15), 83(12), 69(18), 57(35), 55(13), 41(12), 29(10)

### [Example 13 (reference example)]

### ((2-(2-(3,3-Dimethylcyclohexyl)propoxy)ethoxy)methyl)benzene

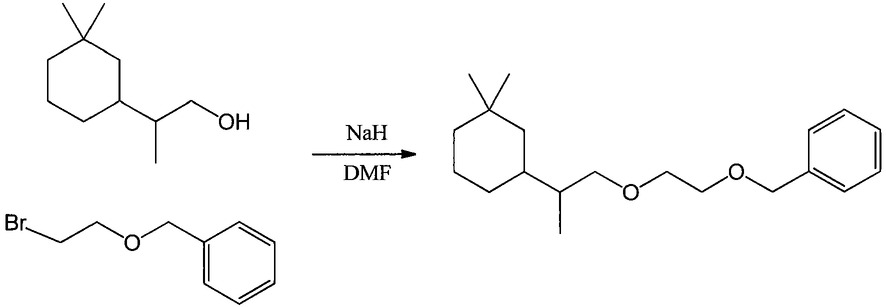

Under a nitrogen atmosphere, a mixed solution of dimethylformamide (DMF, 5 ml) and sodium hydride (370 mg, 9.26 mmol) was cooled to 0°C and 2-(3,3-dimethylcyclohexyl)propan-1-ol (1.05 g, 6.17 mmol) was added dropwise thereto while keeping the mixture at 5°C or less, followed by stirring for 15 minutes. Then, benzyl 2-bromoethyl ether (1.46 ml, 9.26 mmol) was added dropwise while keeping the mixture at 5°C or less and, after dropwise addition, the mixture was warmed to 40°C and stirred for 2 hours. After addition of 20% aqueous ammonium chloride and hexane, the mixture was stirred and the organic layer was then separated. The organic layer was then washed twice with water. After the solvent was distilled off with a rotary evaporator, the residue was purified by column chromatography (silica gel, hexane:ethyl acetate = 9:1) to obtain ((2-(2-(3,3-dimethylcyclohexyl)propoxy)ethoxy)-methyl)benzene (589 mg, 1.85 mmol, yield: 31%).

### [Example 14 (reference example)]

### 2-(2-(3 -3 -Dimethylcyclohexyl)propoxy)ethan-1-ol

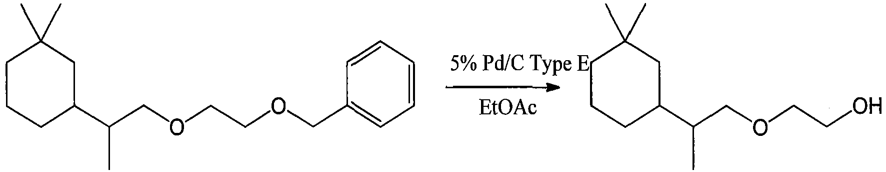

Under a nitrogen atmosphere, to a mixed solution of the resulting ((2-(2-(3,3-dimethylcyclohexyl)propoxy)ethoxy)methyl)benzene (589 mg, 1.85 mmol) and ethyl acetate (6 ml), 5% Pd/C Type E (29.5 mg, 5 wt%) was added, and the reaction vessel was purged with hydrogen, followed by stirring at 15°C for 3 hours. Then, the reaction mixture was filtered through celite, and the solvent was distilled off with a rotary evaporator to obtain 2-(2-(3,3-dimethylcyclohexyl)propoxy)ethan-1-ol (201 mg, 0.94 mmol, yield: 51%) as a crude product.

### [Example 15]

### 2-(2-(3,3 -Dimethylcyclohexyl)propoxy)ethyl propionate

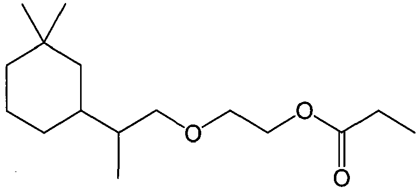

The same procedures as shown in Example 2 were repeated to synthesize the titled compound, except that the alcohol synthesized in Example 14 was used instead of 2-(3,3 -dimethylcyclohexyl)propanonate.

¹H-NMR (400 MHz, CDCl₃): 0.84~0.91(m, 10H), 1.04(t/d, J = 16.9, 7.30 Hz, 1H), 1.15(t, 7.60 Hz, 3H), 1.23~1.29(m, 2H), 1.30~1.36(m, 1H), 1.36(m, 1H), 1.46~1.63(m, 4H), 2.36(q, J =7.60 Hz, 2H), 3.24~3.27(m, 1H), 3.39~3.46(m, 1H), 3.57~3.65(m, 2H), 4.22(t, J = 5.23 Hz, 2H) (diastereomer 1:1 mixture)

GC/MS (m/e):
Isomer 1: 271(1), 196(2), 181(20), 167(1), 153(22), 137(25), 119(20), 101(85), 97(90), 83(70), 69(82), 57(100), 41(35), 29(28)
Isomer 2: 271(1), 213(1), 196(2), 181(22), 163(1), 152(28), 137(30), 119(23), 101(72), 97(95), 93(68), 69(85), 57(100), 41(32), 29(28)

### [Example 16]

### 2-(2-(3,3-Dimethylcyclohexyl)propoxy)ethyl cyclopropanecarboxylate

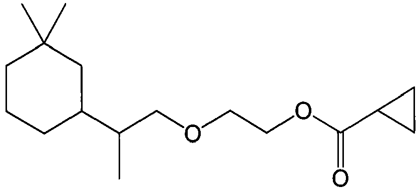

The same procedures as shown in Example 4 were repeated to synthesize the titled compound, except that the alcohol synthesized in Example 14 was used instead of 2-(3,3 -dimethylcyclohexyl)propanonate.

¹H-NMR (400 MHz, CDCl₃): 0.81~0.90 (m, 11H), 1.01(d/t, J = 7.35, 4.45 Hz, 2H), 1.03~1.08(d/d, J = 13.6, 4.55 Hz, 1H), 1.24~1.36(m, 5H), 1.36~1.44(m, 1H), 1.47~1.67(m, 4H), 3.23~3.28(m, 1H), 3.40~3.46(m, 1H), 3.57~3.65(m, 2H), 4.22(t, J = 4.65 Hz, 2H) (diastereomer 1:1 mixture)

GC/MS (m/e):
Isomer 1: 196(1), 181(7), 163(1), 150(10), 123(5), 109(20), 97(35), 83(28), 71(10), 69(100), 55(30), 41(38), 39(8), 29(5)
Isomer 2: 196(1), 181(7), 163(1), 153(11), 137(10), 123(5), 109(20), 97(35), 83(28), 71(10), 69(100), 55(30), 41(38), 39(8), 29(5)

### [Example 17]

### 4-Oxopentyl 2-(3,3 -dimethylcyclohexyl)propanonate

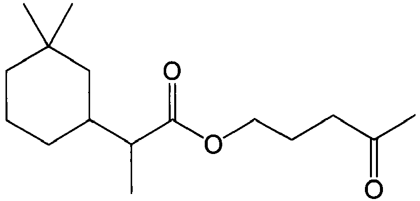

The same procedures as shown in Example 1 were repeated to synthesize the titled compound, except that 5-hydroxy-2-pentanone was used instead of ethylene glycol.

¹H-NMR (400 MHz, CDCl₃): 0.75~0.83(m, 1H), 0.88(s, 6H), 1.01(m, 1H), 1.09(t, J =7.30 Hz, 3H), 1.21~1.47(m, 4H), 1.52~1.64(m, 2H), 1.66~1.75(m, 1H), 1.92(quint. J = 6.85 Hz, 2H), 2.13~2.21(m, 1H), 2.16(s, 3H), 2.52(t, J = 7.20 Hz, 2H), 4.02-4.13(m, 2H) (diastereomer 1:1 mixture)

GC/MS (m/e):
Isomer 1: 268(M⁺, 1), 250(1), 225(1), 211(1), 185(1), 167(1), 158(12), 137(3), 123(2), 95(10), 85(100), 69(15), 55(15), 43(42), 29(3)
Isomer 2: 268(M⁺, 1), 250(1), 225(1), 211(1), 185(1), 167(1), 158(12), 137(3), 123(2), 95(10), 85(100), 69(15), 55(15), 43(42), 29(3)

### [Example 18]

The compounds synthesized in Examples 2 to 12 and 15 to 17 described above were evaluated for the intensity of their scent. As a result of the sensory test made by 3 expert panelists with more than 3 years of experience, the intensity was evaluated in 5 levels, i.e., 5: very strong, 4: strong, 3: normal, 2: weak, and 1: very weak.

[Table 1]

**Table 1**

| Example No. | Structural formula | Aroma quality | Musk intensity (evaluated in 5 levels) |
|---|---|---|---|
| 2 | 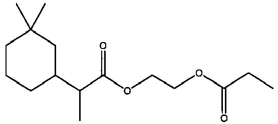 | Musk, powdery, floral | 5 |
| 2a | 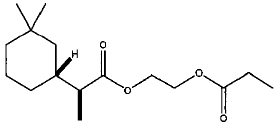 | Musk, powdery, floral | 5 |
| 2b | 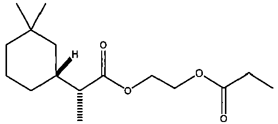 | Very weak musk | 1 |
| 3 | 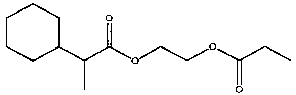 | Musk | 4 |
| 4a | 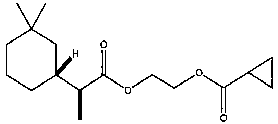 | Musk | 4 |
| 4b | 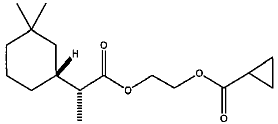 | Musk | 1 |
| 5 | 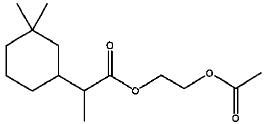 | Musk | 3 |
| 6a | 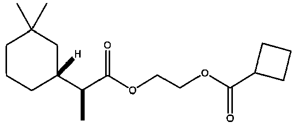 | Musk | 3 |
| 6b | 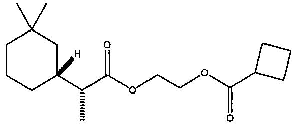 | Musk | 1 |
| 7 | 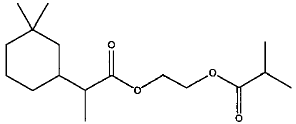 | Musk | 1 |
| 8 | 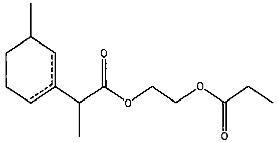 | Musk | 2 |
| 9 | 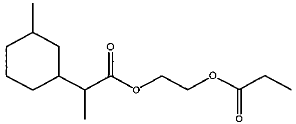 | Musk | 3 |
| 10 | 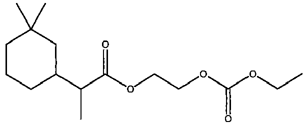 | Musk | 3 |
| 11 | 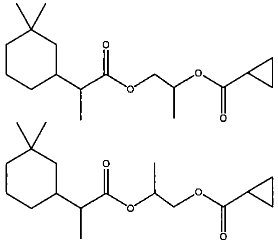 | Musk | 1 |
| 12 | 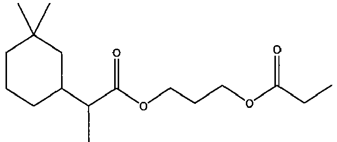 | Musk | 1 |
| 15 | 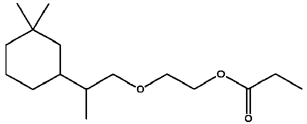 | Musk | 3 |
| 16 | 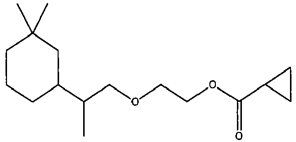 | Musk | 3 |
| 17 | 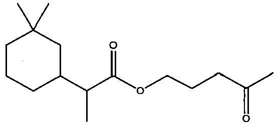 | Musk | 2 |

### [Example 19] Fruity perfume composition

In accordance with the formulation shown in Table 2 below, a fruity perfume composition was prepared. A sensory test was made by 4 expert panelists with more than 5 years of experience, and all of these 4 panelists judged that the fruity perfume composition containing the compound of Example 2, 2a or 3 well diffused a clean and soft scent and was highly palatable and excellent in aroma quality.

[Table 2]

**Table 2**

| INGREDIENT | CONTENT (g) |
|---|---|
| AMBROXAN^{®} (Kao Corporation, Japan) | 5 |
| L-CARVONE | 2 |
| CASSIS BASE | 5 |
| COUMARIN (Fimenich) | 2 |
| CYCLAPROP | 20 |
| DIPROPYLENE GLYCOL (DPG) | 60 |
| GALAXOLIDE | 100 |
| GERANYL ACETATE | 10 |
| HEDIONE^{®} (Firmenich) | 100 |
| HEXYL ACETATE | 10 |
| HEXYL CINNAMIC ALDEHYDE (Kao Corporation, Japan) | 65 |
| HEXYL SALICYLATE (Givaudan) | 25 |
| L-CYTROLE | 30 |
| LILIAL^{®} (Givaudan) | 75 |
| LINALOOL | 45 |
| LINALYL ACETATE | 10 |
| MUSK T^{®} (Takasago International Corporation, Japan ) | 50 |
| Compound of Example 2, 2a or 3 | 10 |
| ORANGE OIL ESSENTIAL OIL | 35 |
| ORBITONE BHT / ISO E SUPER^{®} (Takasago International Corporation, Japan) | 85 |
| RED FRUITS BASE | 60 |
| ROSE BASE | 26 |
| TERPINEOL (International Flavors & Fragrances) | 40 |
| MYRCENOL | 50 |
| TRIPLAL^{®} (International Flavors & Fragrances) | 10 |
| UNDECALACTONE, GAMMA (ALD C-14) | 10 |
| VERDOX^{®} (International Flavors & Fragrances) | 25 |
| WATERY FLOWER ACCORD | 26 |
| WOODY MUSK ACCORD | 9 |
| | |
| TOTAL | 1000 |

### [Example 20] Shampoo

In accordance with the formulation shown below, a shampoo (100 g) was prepared by being perfumed with 1.0% of the perfume composition containing the compound of Example 2, 2a or 3. A sensory test was made by 4 expert panelists with more than 5 years of experience, and all of these 4 panelists judged that this shampoo was clearly found to have a highly palatable and clean fruity scent, and was excellent in aroma quality.

| Formulation (ingredients) | Content (g) |
|---|---|
| Polyoxyethylenelauryl ether sodium sulfate | 14.00 |
| Lauramidopropyl betaine | 4.00 |
| Coconut oil fatty acid diethanolamide | 3.00 |
| Cationic cellulose | 0.50 |
| Ethylene glycol distearate | 1.00 |
| Ethyl parahydroxybenzoate | 0.25 |
| Citric acid | q.s. |
| Perfume composition prepared in Example 19 | 1.00 |
| Purified water | balance |
| Total | 100.00 |

### [Example 21 (reference example)]

### ((2-(1-(3,3-Dimethylcyclohexyl)ethoxy)ethoxy)methyl)benzene

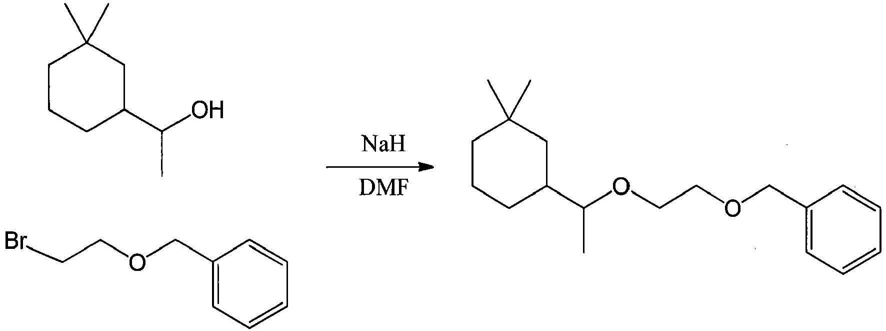

Under a nitrogen atmosphere, a mixed solution of dimethylformamide (DMF, 19 ml) and sodium hydride (1.21 g, 30.2 mmol) was cooled to 0°C and 1-(3,3-dimethylcyclohexyl)ethan-1-ol (3.16 g, 20.2 mmol) was added dropwise thereto while keeping the mixture at 5°C or less, followed by stirring for 15 minutes. Then, benzyl 2-bromoethyl ether (4.5 ml, mmol) was added dropwise while keeping the mixture at 5°C or less and, after dropwise addition, the mixture was warmed to 40°C and stirred for 2 hours. After addition of 20% aqueous ammonium chloride and hexane, the mixture was stirred and the organic layer was then separated. The organic layer was then washed twice with water. After the solvent was distilled off with a rotary evaporator, the residue was purified by column chromatography (silica gel, hexane:ethyl acetate = 9:1) to obtain ((2-(1-(3,3-dimethylcyclohexyl)ethoxy)ethoxy)methyl)benzene (1.33 g, 4.58 mmol, yield: 15%).

¹H-NMR (400 MHz, CDCl₃): 0.87(s, 3H), 0.90(s, 3H), 1.09(d, J = 5.20 Hz, 3H), 1.25~1.52(m, 4H), 1.52~1.61(m, 3H), 1.62~1.73(m, 2H), 3.13(d/t, J = 10.0, 5.20 Hz, 1H), 3.53(m, 1H), 3.62(t/d, J = 8.4, 3.6 Hz, 2H), 3.68(m, 1H), 4.58(s, 2H), 7.27(m, 1H), 7.35(m, 5H)

¹³C-NMR (125 Hz, CDCl₃): 138.50(s), 128.29(d), 128.29(d), 127.62(d), 127.62(d), 127.46(d), 80.58(d), 73.16(t), 69.87(t), 68.22(t), 41.98(t), 39.39(t), 38.88(d), 33.63(q), 30.60(s), 28.23(t), 24.67(q), 24.08(t), 16.45(q)
GC/MS (m/e): 290(1), 213(1), 199(1), 179(3), 155(1), 151(33), 137(2), 123(5), 107(13), 91(100), 83(11), 69(14), 55(11), 41(8), 26(2)

### [Example 22 (reference example)]

### 2-(1-(3,3-Dimethylcyclohexyl)ethoxy)ethan-1-ol

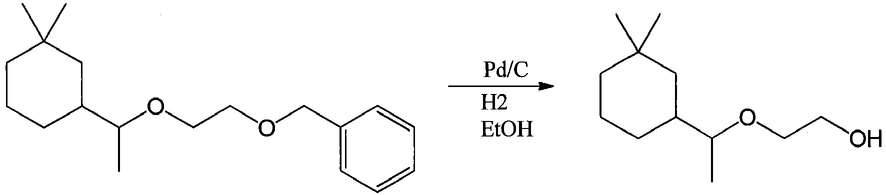

Under a nitrogen atmosphere, to a mixed solution of the resulting ((2-(1-(3,3-dimethylcyclohexyl)ethoxy)ethoxy)methyl)benzene (1.33 g, 4.58 mmol) and ethyl acetate (7 ml), a 5% Pd/carbon catalyst (28.3 mg, 2.1 wt%) was added, and the reaction vessel was purged with hydrogen, followed by stirring at 15 °C for 5 hours. Then, the reaction mixture was filtered through celite, and the solvent was distilled off with a rotary evaporator to obtain 2-(1-(3,3-dimethylcyclohexyl)ethoxy)ethan-1-ol (312 mg, 1.56 mmol, yield: 34%) as a crude product.

¹H-NMR (400 MHz, CDCl₃): 0.85(t, J = 12.6 Hz, 1H), 0.88(s, 3H), 0.90(s, 3H), 1.06(t/d, J = 13.2, 4.05 Hz, 1H), 1.10(d, J = 6.30 Hz, 3H), 1.34(m, 1H), 1.42(d/t, J = 13.5, 3.50 Hz, 1H), 1.49(m, 1H), 1.53~1.66(m, 3H), 2.12(d/d, J = 6.20, 6.20 Hz, 1H), 3.15(d/t, J = 12.2, 6.15 Hz, 1H), 3.42(m, 1H), 3.61(m, 1H), 3.71(d/d/d, J = 5.90, 5.35, 1.05 Hz, 2H)

¹³C-NMR (125 Hz, CDCl₃): 80.43(d), 69.70(t), 62.13(t), 41.87(t), 39.32(t), 38.91(d), 33.60(q), 30.59(s), 28.27(t), 24.65(q), 22.11(t), 16.40(q)
GC/MS (m/e): 185(1), 155(1), 137(3), 123(8), 109(2), 95(4), 89(100), 81(7), 69(13), 57(3), 55(15), 45(94), 39(4), 29(7)

### [Example 23]

### 2-(1-3,3-Dimethylcyclohexyl)ethoxy)ethyl propionate

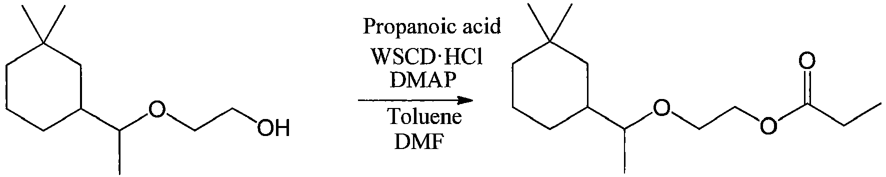

The same procedures as shown in Example 2 were repeated to synthesize the titled compound, except that 2-(1-(3,3-dimethylcyclohexyl)ethoxy)ethan-1-ol was used instead of 2-(propionyloxy)ethyl 2-(3,3-dimethylcyclohexyl)propanonate.

¹H-NMR (400 MHz, CDCl₃): 0.81(t, J = 12.8 Hz, 1H), 0.86(t/d, J = 12.4, 4.10 Hz, 1H), 0.88(s, 3H), 0.91 (s, 3H), 1.05(d/d/d, J = 3.85, 13.3 Hz, 1H), 1.09(d, J = 7.60 Hz, 3H), 1.15(t, J = 7.60 Hz, 3H), 1.34(m, 1H), 1.41(d/d//t, J = 26.8, 13.4, 3.70 Hz, 1H), 1.51~1.60(m, 2H), 1.56(s, 1H), 1.63(m, 1H), 2.36(q. J = 7.60 Hz, 2H), 3.1(quint, J = 6.20 Hz, 1H), 3.55(d/d/d, J = 11.0, 5.90, 4.40 Hz, 1H), 3.69(d/d/d, J = 11.2, 4.20, 5.20 Hz, 1H), 4.20(d/d/d, J = 5.98, 4.25, 1.25 Hz, 2H)

¹³C-NMR (125 Hz, CDCl₃): 174.52(s), 80.69(d), 66.72(t), 63.85(t), 41.98(t), 39.34(t), 38.93(d), 33.63(q), 30.58(s), 28.33(t), 27.58(t), 24.68(q), 22.16(t), 16.58(q), 9.12(q)

GC/MS (m/e): 182(1), 155(3), 136(6), 111(2), 101(100), 95(3), 81(7), 69(9), 57(25), 55(8), 41(6), 27(6)

### [Example 24]

### 2-(1-(3,3 -Dimethylcyclohexyl)ethoxy)ethyl cyclobutanecarboxylate

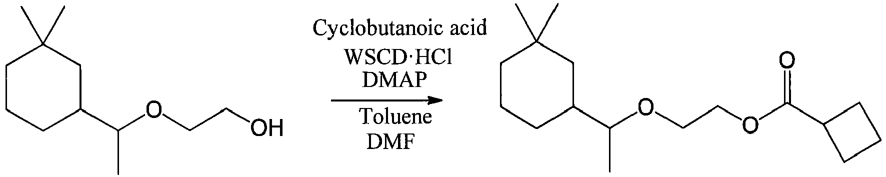

The same procedures as shown in Example 2 were repeated to synthesize the titled compound, except that 2-(1-(3,3-dimethylcyclohexyl)ethoxy)ethan-1-ol was used instead of 2-(propionyloxy)ethyl 2-(3,3-dimethylcyclohexyl)propanonate and cyclobutanoic acid was used instead of propionic acid.

¹H-NMR (400 MHz, CDCl₃): 0.81(t, J = 10.4 Hz, 1H), 0.88(s, 3H), 0.91(s, 3H), 0.94~0.98(m, 1H), 1.05(t/d, J = 10.8, 3.2 Hz, 1H), 1.08(d, J = Hz, 3H), 1.33(m, 1H), 1.41(d/d/t, J = 10.6, 10.4, 3.2 Hz, 1H), 1.51~1.60 (m, 2H), 1.60~1.65(m, 2H), 1.91(m, 1H), 1.96(m, 1H), 2.21(m, 2H), 2.31(m, 2H), 3.10(t/t, J = 10.0, 4.8 Hz, 1H), 3.16(d/t, J = 13.6, 7.2 Hz, 1H), 3.55(m, 1H), 3.69(m, 1H), 4.20(t, J = 4.0 Hz, 2H)

¹³C-NMR (125 Hz, CDCl₃): 175.51(s), 80.62(d), 66.75(t), 63.81(t), 41.94(t), 39.35(t), 38.97(d), 38.12(d), 33.62(q), 30.58(s), 28.36(t), 25.28(t), 25.28(t), 24.68(q), 22.16(t), 22.16(t), 18.42(t), 16.57(q)

GC/MS (m/e): 283(1), 237(1), 199(1), 171(4), 155(3), 140(2), 127(100), 123(7), 99(12), 97(2), 83(25), 69(13), 55(33), 25(2)

### [Example 25]

### 2-(1-(3,3-Dimethylcyclohexyl)ethoxy)ethyl isobutyrate

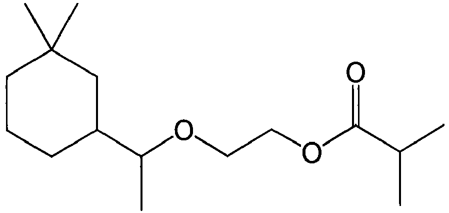

The same procedures as shown in Example 2 were repeated to synthesize the titled compound, except that 2-(1-(3,3-dimethylcyclohexyl)ethoxy)ethan-1-ol was used instead of 2-(propionyloxy)ethyl 2-(3,3-dimethylcyclohexyl)propanonate and isopropionic acid was used instead of propionic acid.

¹H-NMR (400 MHz, CDCl₃): 0.81(s, 3H), 0.84(s, 3H), 0.98(t/d, J = 13.2, 4.25 Hz, 1H), 1.02(d, J = 6.25 Hz, 3H), 1.10(s, 3H), 1.12(s, 3H), 1.17~1.39(m, 3H), 1.44~1.52(m, 4H), 1.56(m, 1H), 2.51(t/t, J = 14.0, 7.00 Hz, 1H), 3.02(d/d/d, J = 6.15, 6.15, 12.4 Hz, 1H), 3.48(m, 1H), 3.62(m, 1H), 4.12(t/d, J = 4.85, 1.50 Hz, 2H)

¹³C-NMR (125 Hz, CDCl₃): 177.15(s), 80.57(d), 66.75(t), 63.80(t), 41.94(t), 39.34(t), 38.98(d), 33.97(d), 33.60(q), 30.57(s), 28.36(t), 24.66(q), 22.14(t), 18.99(q), 16.56(q), 14.10(q)

GC/MS (m/e): 271(1), 225(1), 199(1), 182(2), 167(1), 159(8), 139(23), 124(3), 115(100), 109(4), 95(7), 83(33), 71(34), 55(25), 43(65), 29(5)

### [Example 26 (reference example)]

### 3-(1-3,3-Dimethylcyclohexyl)ethoxy)propyl acetate

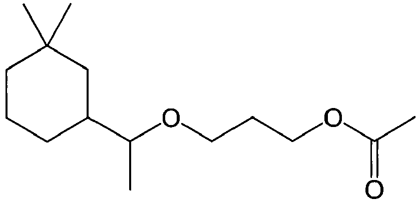

The same procedures as shown in Example 2 were repeated to synthesize the titled compound, except that 3-(1-(3,3-dimethylcyclohexyl)ethoxy)propan-1-ol was used instead of 2-(propionyloxy)ethyl 2-(3,3-dimethylcyclohexyl)propanonate and acetic acid was used instead of propionic acid.

¹H-NMR (400 MHz, CDCl₃): 0.74~0.90(m, 2H), 0.87(s, 3H), 0.90(s, 3H), 1.06(d, J = Hz, 3H), 1.28(m, 1H), 1.33(m, 1H), 1.35~1.45(m, 1H), 1.48(m, 1H), 1.50~1.59(m, 2H), 1.62(m, 1H), 1.87(t/t/d, J = 12.7, 6.30, 0.85 Hz, 2H), 2.05(s, 3H), 3.05(t/d, J = 12.3, 6.15 Hz, 1H), 3.37(m, 1H), 3.56(m, 1H), 4.16(t/d, J = 6.50, 0.45 Hz, 2H) (diastereomer 1:1 mixture)

¹³C-NMR (125 Hz, CDCl₃): 171.07(s), 80.19(d), 65.06(t), 61.93(t), 41.93(t), 39.34(t), 38.93(d), 33.59(q), 30.57(s), 29.38(t), 28.29(t), 24.69(q), 22.15(t), 20.94(q), 16.48(q)

GC/MS (m/e):
Isomer 1: 257(1), 241(1), 196(1), 181(1), 155(3), 145(12), 137(4), 123(13), 101(100), 95(5), 73(28), 69(15), 55(15), 43(63), 29(5)
Isomer 2: 257(1), 241(1), 196(1), 181(1), 155(3), 145(12), 124(2), 123(12), 101(100), 95(5), 73(28), 69(15), 55(15), 43(65), 29(5)

### [Example 27 (reference example)]

### 2-(1-3,3-Dimethylcyclohexyl)ethoxy)ethyl cyclohex-1-en-1-carboxylate

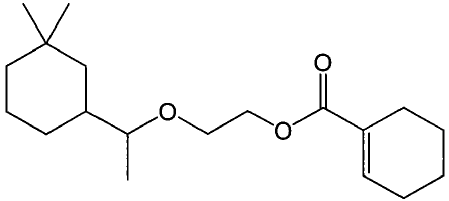

The same procedures as shown in Example 2 were repeated to synthesize the titled compound, except that 2-(1-(3,3-dimethylcyclohexyl)ethoxy)ethan-1-ol was used instead of 2-(propionyloxy)ethyl 2-(3,3-dimethylcyclohexyl)propanonate and 1-cyclohexene-1-carboxylic acid was used instead of propionic acid.

¹H-NMR (400 MHz, CDCl₃): 0.80(t, 1H), 0.84(s, 3H), 0.88(s, 3H), 1.05(t/d, J = Hz, 1H), 1.08(d, J = Hz, 3H), 1.34(m, 1H), 1.60(m, 1H), 1.50~1.71(m, 7H), 2.18(m, 2H), 2.27(m, 2H), 2.32(m, 2H), 3.10(d/t, J = Hz, 1H), 3.58(m, 1H), 3.73(m, 1H), 4.24(m, 2H), 7.02(m, 1H)

¹³C-NMR (125 Hz, CDCl₃): 167.56(s), 139.88(d), 130.25(s), 80.61(d), 66.82(t), 63.71(t), 42.00(t), 39.32(t), 39.02(d), 33.59(q), 30.52(s), 28.41(t), 25.75(t), 24.64(q), 24.12(t), 22.04(t), 21.78(t), 21.17(t), 16.66(q)

GC/MS (m/e): 309(1), 220(1), 197(3), 170(2), 154(17), 153(100), 139(19), 123(6), 109(52), 97(4), 81(27), 69(16), 55(15), 41(14), 26(2)

### [Example 28 (reference example)]

### 3-(1-3,3-Dimethylcyclohexyl)ethoxy)propyl cyclopropanecarboxylate

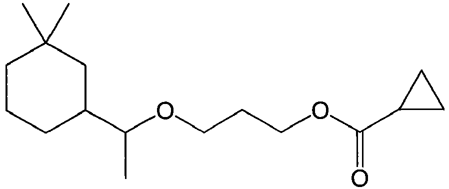

The same procedures as shown in Example 2 were repeated to synthesize the titled compound, except that 3-(1-(3,3-dimethylcyclohexyl)ethoxy)propan-1-ol was used instead of 2-(propionyloxy)ethyl 2-(3,3-dimethylcyclohexyl)propanonate and cyclopropanoic acid was used instead of propionic acid.

¹H-NMR (400 MHz, CDCl₃): 0.74~0.87(m, 3H), 0.87(s, 3H), 0.90(s, 3H), 0.98(m, 2H), 1.06(d, J = 6.25 Hz, 3H), 1.23~1.36(m, 3H), 1.41(m, 1H), 1.18(m, 1H), 1.50~1.64(m, 3H), 1.87(d/t, J = 12.7, 6.35 Hz, 2H), 3.06(t/d, J = 12.8, 6.40 Hz, 1H), 3.37(m, 1H), 3.57(m, 1H), 4.17(t, J = 6.45 Hz, 2H) (diastereomer 1:1 mixture)

¹³C-NMR (125 Hz, CDCl₃): 174.81(s), 80.09(d), 65.06(t), 61.87(t), 41.83(t), 39.36(t), 39.00(d), 33.60(q), 30.57(s), 29.47(t), 28.83(t), 28.29(t), 24.70(q), 22.16(t), 16.49(q), 12.84(d), 8.25(t)

GC/MS (m/e):
Isomer 1: 283(1), 267(1), 213(1), 186(1), 171(4), 143(8), 127(100), 123(12), 99(7), 83(15), 69(83), 55(17), 41(29), 25(3)
Isomer 2: 283(1), 267(1), 213(1), 186(1), 171(4), 143(8), 127(100), 123(12), 99(7), 83(15), 69(88), 55(17), 41(29), 25(3)

### [Example 29]

### 2-(1-(3,3-Dimethylcyclohexyl)ethoxy)ethyl cyclopropanecarboxylate

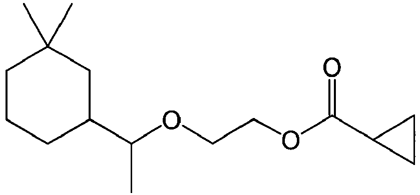

The same procedures as shown in Example 2 were repeated to synthesize the titled compound, except that 2-(1-(3,3-dimethylcyclohexyl)ethoxy)ethan-1-ol was used instead of 2-(propionyloxy)ethyl 2-(3,3-dimethylcyclohexyl)propanonate and cyclopropanoic acid was used instead of propionic acid.

¹H-NMR (400 MHz, CDCl₃): 0.78~0.88(m, 4H), 0.88(s, 3H), 0.91(s, 3H), 1.01(m, 2H), 1.06(t/d, J = 13.4, 4.05 Hz, 1H), 1.10(d, J = 10.2 Hz, 3H), 1.34(m, 1H), 1.42(d/d/t, J = 26.8, 13.4, 3.70 Hz, 1H), 1.51~1.67(m, 5H), 3.11(d/t, J = 12.3, 6.15 Hz, 1H), 3.55(m, 1H), 3.69(m, 1H), 4.20(t, J = 5.40 Hz, 2H)

¹³C-NMR (125 Hz, CDCl₃): 174.88(s), 80.65(d), 66.71(t), 63.93(t), 41.95(t), 39.33(t), 38.91(d), 33.60(q), 30.56(s), 28.31(t), 24.66(q), 22.14(t), 16.57(q), 12.89(d), 8.41(t), 8.40(t)

GC/MS (m/e): 182(1), 155(3), 139(7), 123(5), 113(100), 109(2), 97(3), 84(2), 83(10), 69(25), 55(8), 41(12), 29(2)

### [Example 30]

The compounds synthesized in Examples 23 to 29 described above were evaluated for the intensity of their scent. As a result of the sensory test made by 4 expert panelists with more than 3 years of experience, the intensity was evaluated in 5 levels, i.e., 5: very strong, 4: strong, 3: normal, 2: weak, and 1: very weak.

### [Table 3]

**Table 3**

| Example No. | Structural formula | Aroma quality | Musk intensity (evaluated in 5 levels) |
|---|---|---|---|
| 24 | 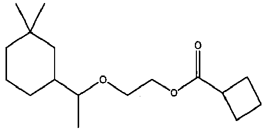 | Musk, powdery, soft | 5 |
| 23 | 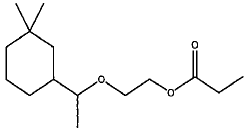 | Musk, fruity, aquatic | 4 |
| 25 | 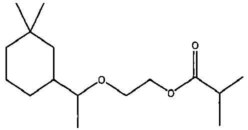 | Musk, herbal, fruity | 3 |
| 29 | 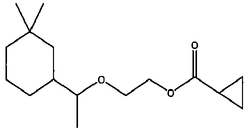 | Musk, green, fatty | 2 |
| 26 (reference example) | 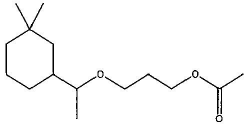 | Musk | 1 |
| 27 (reference example) | 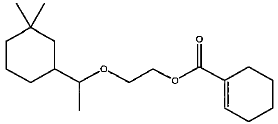 | Musk | 1 |
| 28 (reference example) | 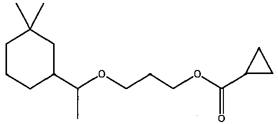 | Musk | 1 |

### [Example 31] Shampoo and hairpiece

A shampoo (1.0 g) was prepared by being perfumed with 0.5% of the compound of Example 24 or Helvetolide^{®} (Firmenich). The resulting shampoos were confirmed for the effect of their scent during foaming and in wet or dry state in a hairpiece by 6 expert panelists with more than 3 years of experience.

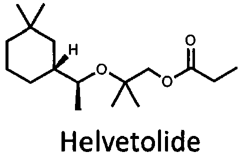

As a result, 3 of the expert panelists responded that Helvetolide was more fragrant during foaming, but was equally or less fragrant than Example 24 in wet state. In dry state, Example 24 was evaluated to give the strongest lingering scent by all of the 6 expert panelists.

### [Example 32] Softener

A softener (0.2 g) was prepared by being perfumed with 0.5% of the compound of Example 24 or Helvetolide. A sensory test was made by 4 expert panelists with more than 3 years of experience, and all of these 4 expert panelists judged that Example 24 was clearly found to give a highly intense musk sensation comparable to that in smelling strips, and was excellent in aroma quality.

### INDUSTRIAL APPLICABILITY

The compound of the present invention can be used as a perfume material capable of imparting a musk-like scent. Products can be scented with a perfume composition comprising the compound of the present invention, so that the commercial value of the products can be increased.

## Claims

1. A compound represented by the following general formula: 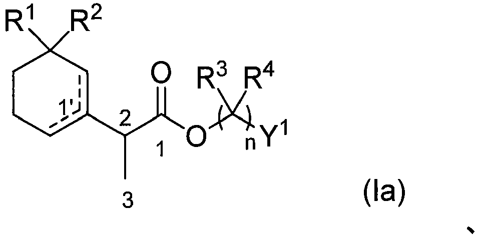 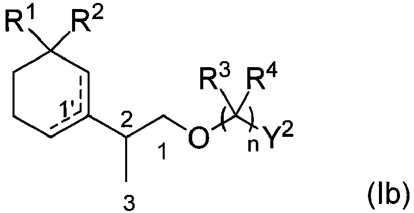 or 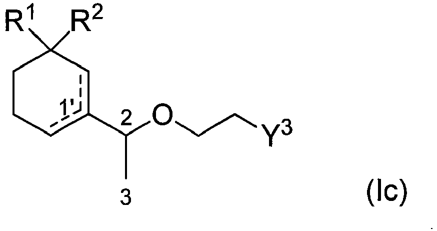 wherein
R¹ and R² are each independently a hydrogen atom or a methyl group,
n is 2 or 3,
two or more R³ and R⁴ are each independently a hydrogen atom or a methyl group,
Y¹ is -O-CO-Z, -O-CO-O-Z, -CO-Z, -CO-O-Z or -CH(Z¹)-CO-Z² , Y² is -O-CO-Z or -O-CO-O-Z, and Y³ is -O-CO-Z³, where Z is a linear or branched alkyl group containing 1 to 5 carbon atoms or a cycloalkyl group containing 3 to 5 carbon atoms, Z¹ is a hydrogen atom, a methyl group or an ethyl group, Z² is a linear or branched alkyl group containing 1 to 3 carbon atoms, and Z³ is a linear or branched alkyl group containing 1 to 5 carbon atoms or a cycloalkyl group containing 3 to 5 carbon atoms, and
the adjacent two ring-constituting double lines, each consisting of a solid line and a dotted line, are each independently a double bond or a single bond, except for the case where both are double bonds.

2. The compound according to claim 1, which is represented by the following general formula: 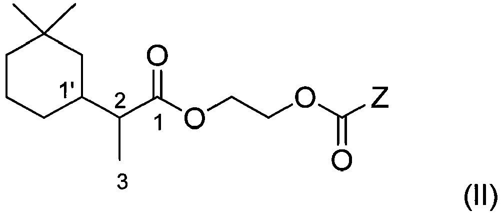 wherein Z is a linear or branched alkyl group containing 1 to 5 carbon atoms or a cycloalkyl group containing 3 to 5 carbon atoms.

3. The compound according to claim 1 or 2, which is represented by the following structural formula.

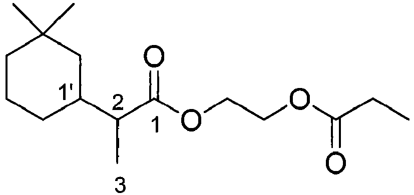

4. The compound according to claim 3, wherein the relative configuration at the 1'- and 2-positions is (R^{∗},S^{∗}) or (R^{∗},R^{∗}) or a mixture of (R^{∗},S^{∗}) and (R^{∗},R^{∗}).

5. The compound according to claim 3, wherein the relative configuration at the 1'- and 2-positions is (R^{∗},S^{∗}).

6. A perfume composition containing at least one compound according to any one of claims 1 to 5.

7. A musk perfume composition containing at least one compound according to any one of claims 1 to 5.

8. A product scented with the perfume composition according to claim 6 or 7.

9. The product according to claim 8, wherein the product is one selected from fragrance products, perfumery and cosmetic products, basic cosmetics, make-up cosmetics, hair cosmetics, sunburn cosmetics, medicated cosmetics, hair care products, soaps, body washes, bath preparations, fabric detergents, fabric softeners, detergents, kitchen detergents, bleaching agents, aerosols, air fresheners, repellents and miscellaneous goods.

10. A method for enhancing or modulating the scent of a perfume composition, which comprises adding at least one compound according to any one of claims 1 to 5.

## Patentansprüche

1. Verbindung, dargestellt durch die folgende allgemeine Formel: 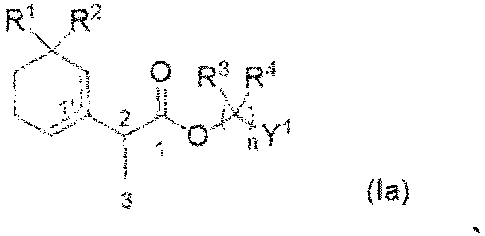 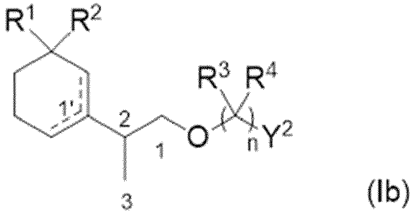 oder 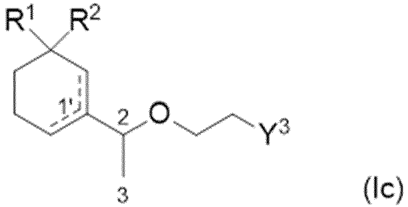 wobei
R¹ und R² jeweils unabhängig voneinander ein Wasserstoffatom oder eine Methylgruppe darstellen,
n 2 oder 3 ist,
zwei oder mehr von R³ und R⁴ jeweils unabhängig voneinander ein Wasserstoffatom oder eine Methylgruppe darstellen,
Y¹ -O-CO-Z, -O-CO-O-Z, -CO-Z, -CO-O-Z oder -CH(Z¹)-CO-Z² darstellt, Y² -O-CO-Z oder -O-CO-O-Z darstellt, und Y³ -O-CO-Z³ darstellt, wobei Z eine lineare oder verzweigte Alkylgruppe mit 1 bis 5 Kohlenstoffatomen oder eine Cycloalkylgruppe mit 3 bis 5 Kohlenstoffatomen darstellt, Z¹ ein Wasserstoffatom, eine Methylgruppe oder eine Ethylgruppe darstellt, Z² eine lineare oder verzweigte Alkylgruppe mit 1 bis 3 Kohlenstoffatomen darstellt und Z³ eine lineare oder verzweigte Alkylgruppe mit 1 bis 5 Kohlenstoffatomen oder eine Cycloalkylgruppe mit 3 bis 5 Kohlenstoffatomen darstellt, und
die beiden benachbarten ringbildenden Doppellinien, die jeweils aus einer durchgezogenen Linie und einer gestrichelten Linie bestehen, jeweils unabhängig voneinander eine Doppelbindung oder eine Einfachbindung sind, ausgenommen der Fall, dass beide Doppelbindungen sind.

2. Verbindung gemäß Anspruch 1, die durch die folgende allgemeine Formel dargestellt wird: 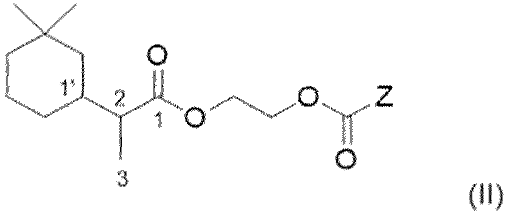 wobei Z eine lineare oder verzweigte Alkylgruppe mit 1 bis 5 Kohlenstoffatomen oder eine Cycloalkylgruppe mit 3 bis 5 Kohlenstoffatomen darstellt.

3. Verbindung gemäß Anspruch 1 oder 2, die durch die folgende Strukturformel dargestellt wird.

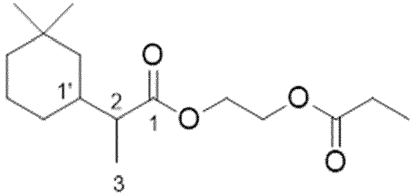

4. Verbindung gemäß Anspruch 3, wobei die relative Konfiguration an den Positionen 1' und 2 (R*, S*) oder (R*, R*) oder eine Mischung aus (R*, S*) und (R*, R*) ist.

5. Verbindung gemäß Anspruch 3, wobei die relative Konfiguration an den Positionen 1' und 2 (R*, S*) ist.

6. Parfümzusammensetzung, enthaltend mindestens eine Verbindung gemäß einem der Ansprüche 1 bis 5.

7. Moschusparfümzusammensetzung, enthaltend mindestens eine Verbindung gemäß einem der Ansprüche 1 bis 5.

8. Produkt, das mit der Parfümzusammensetzung gemäß Anspruch 6 oder 7 parfümiert ist.

9. Produkt gemäß Anspruch 8, wobei das Produkt eines ist, ausgewählt aus Duftstofferzeugnissen, Parfümerie- und Kosmetikprodukten, Basiskosmetika, Make-up-Kosmetika, Haarkosmetika, Sonnenbrandkosmetika, medizinischen Kosmetika, Haarpflegeprodukten, Seifen, Körperwaschmitteln, Badezusätzen, Textilwaschmitteln, Weichspülern, Reinigungsmitteln, Küchenreinigern, Bleichmitteln, Aerosolen, Lufterfrischern, Repellenzien und anderen Produkten.

10. Verfahren zur Verstärkung oder Modulation eines Dufts einer Parfümzusammensetzung, das die Zugabe mindestens einer Verbindung gemäß einem der Ansprüche 1 bis 5 umfasst.

## Revendications

1. Composé représenté par la formule générale suivante : 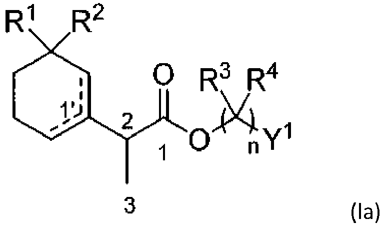 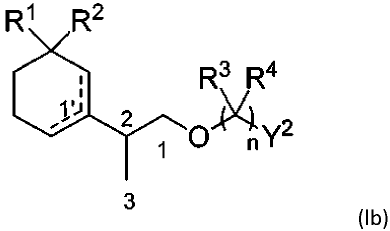 ou 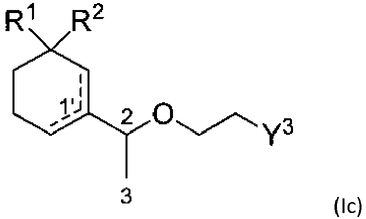 dans lequel
R¹ et R² sont chacun indépendamment un atome d'hydrogène ou un groupe méthyle,
n est 2 ou 3,
deux R³ et R⁴ ou plus sont chacun indépendamment un atome d'hydrogène ou un groupe méthyle,
Y¹ est -O-CO-Z, -O-CO-O-Z, -CO-Z, -CO-O-Z ou -CH(Z¹)-CO-Z², Y² est -O-CO-Z ou -O-CO-O-Z, et Y³ est -O-CO-Z³, où Z est un groupe alkyle linéaire ou ramifié contenant 1 à 5 atomes de carbone ou un groupe cycloalkyle contenant 3 à 5 atomes de carbone, Z¹est un atome d'hydrogène, un groupe méthyle ou un groupe éthyle, Z² est un groupe alkyle linéaire ou ramifié contenant 1 à 3 atomes de carbone, et Z³ est un groupe alkyle linéaire ou ramifié contenant 1 à 5 atomes de carbone ou un groupe cycloalkyle contenant 3 à 5 atomes de carbone, et
les deux lignes doubles adjacentes constituant le cycle, constituées chacune d'un trait plein et d'un trait pointillé, sont chacune indépendamment une double liaison ou une simple liaison, sauf dans le cas où les deux sont des doubles liaisons.

2. Composé selon la revendication 1, qui est représenté par la formule générale suivante : 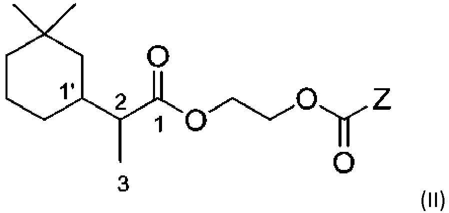 dans lequel Z est un groupe alkyle linéaire ou ramifié contenant 1 à 5 atomes de carbone ou un groupe cycloalkyle contenant 3 à 5 atomes de carbone.

3. Composé selon la revendication 1 ou 2, qui est représenté par la formule structurelle suivante :

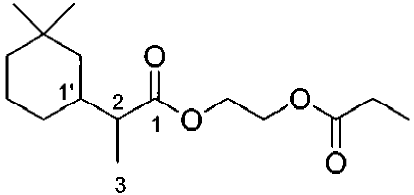

4. Composé selon la revendication 3, dans lequel la configuration relative aux positions 1' et 2 est (R^{∗},S^{∗}) ou (R^{∗},R^{∗}) ou un mélange de (R^{∗},S^{∗}) et (R^{∗},R^{∗}).

5. Composé selon la revendication 3, dans lequel la configuration relative aux positions 1' et 2 est (R^{∗},S^{∗}).

6. Composition de parfum contenant au moins un composé selon l'une quelconque des revendications 1 à 5.

7. Composition de parfum de musc contenant au moins un composé selon l'une quelconque des revendications 1à 5.

8. Produit parfumé avec la composition de parfum selon la revendication 6 ou 7.

9. Produit selon la revendication 8, dans lequel le produit est un sélectionné parmi les produits de fragrance, les produits de parfumerie et cosmétiques, les cosmétiques de base, les cosmétiques de maquillage, les cosmétiques capillaires, les cosmétiques contre les coups de soleil, les cosmétiques médicamenteux, les produits de soins capillaires, les savons, les nettoyants pour le corps, les préparations pour le bain, les détergents pour textiles, les assouplissants pour textiles, les détergents, les détergents de cuisine, les agents de blanchiment, les aérosols, les désodorisants, les répulsifs et des articles divers.

10. Procédé pour renforcer ou moduler l'odeur d'une composition de parfum, qui comprend l'ajout d'au moins un composé selon l'une quelconque des revendications 1 à 5.
